# EUROPEAN PATENT SPECIFICATION

(11) **EP 0 453 560 B1**
(45) Date of publication and mention of the grant of the patent: **26.05.1999**
(21) Application number: 91901084.3
(22) Date of filing: 13.11.1990
(51) Int. Cl.: C12Q 1/68, G01N 33/574, C12N 15/12, A61K 48/00

(54) **LOCALIZATION AND CHARACTERIZATION OF THE WILMS' TUMOR GENE**
LOKALISATION UND CHARAKTERISIERUNG DES WILMS-TUMOR-GENS
LOCALISATION ET CARACTERISATION DU GENE TUMORAL DE WILMS

(30) Priority: 13.11.1989 US 435780
(43) Date of publication of application: 30.10.1991
(73) Proprietor: MASSACHUSETTS INSTITUTE OF TECHNOLOGY, Cambridge, MA 02139 (US)
(72) Inventor: CALL, Katherine, M., Malden, MA 02148 (US); GLASER, Thomas, M., Belmont, MA 02178 (US); ITO, Caryn, Y., Chapel Hill, NC 27514 (US); BUCKLER, Alan, J., Cambridge, MA 02138 (US); PELLETIER, Jerry, Cambridge, MA 02141 (US); HABER, Daniel, A., Cambridge, MA 02139 (US); ROSE, Elise, A., Kensington, CA 94708 (US); HOUSMAN, David, E., Newton, MA 02159 (US)
(74) Representative: Holdcroft, James Gerald, Dr.
(86) International application number: PCT/US90/06629
(87) International publication number: WO 91/07509

(56) References cited:
- CELL. vol. 60, February 1990, CAMBRIDGE,MA US pages 509 - 520; K.M. CALL ET AL: 'Isolation and characterization of a zinc finger polypeptide gene at the human chromosome 11 Wilm's tumor locus'
- NATURE. vol. 343, 22 February 1990, LONDON GB pages 774 - 778; M. GESSLER ET AL.: 'Homozygous deletions in Wilms tumours of a zinc-finger gene identified by chromosome jumping'
- Genomics, Vol. 3 No. 2 issued August 1988, GESSLER et al., "Molecular Mapping and Cloning of the Breakpoints of a chromosome 11p14.1-p13, Deletion Associated with the WAGR Syndrome", pp. 117-123, see Abstract.
- Genomics, Vol. 3, issued July 1988, LEWIS et al. "Homozygous Deletion of a DNA marker from Chromosome 11p13 in Sporadic Wilms Tumor" pp 25-31, see Abstract.
- Cell, Vol. 55, issued 02 December 1988, COMPTON et al., "Long Range Physical Map of the Wilms Tumor-Aniridia Region Human Chromosome 11", pp. 827-836, see Abstract.
- Nature, Vol. 321, No. 6073 issued 26 June 1986 GLASER et al., "The beta-subunit of follicle-stimulating hormone is deleted in patients with aniridia and Wilm's tumor allowing a further definition of the WAGR locus", pp 882-887, see Abstract.
- Proc. Natl. Acad Sci USA, Vol. 84, issued August 1987, PORTEOUS et al., "MRASI- selected chromosome transfer generates markers that colocalize breakpoints and the Wilms tumor gene within band 11p13", pp. 5355-5359, see Abstract.
- Science, Vol. 244, issued 30 June 1989, "GESSLER et al., "Cloning of Breakpoints of a Chromosome Translocation Identifies the AN2 Locus", pp 1575- 1578, see Abstract.

## Description

### Funding

Work described herein was funded by the National Institutes of Health, the Medical Research Council of Canada and the National Cancer Institute.

### Background

Wilms' tumor (WT) is an embryonal malignancy of the kidney which affects approximately 1 in 10,000 infants and young children. Matsunaga, Human Genetics, 57:231-246 (1981). The molecular basis of Wilms' tumor is not well understood.

A subset of Wilms' tumor cases (approximately 2%) occur in association with aniridia (AN2), a defect in the development of the iris, as well as urogenital abnormalities and mental retardation. Miller et al., New Engl. J. Med.*,* 270:922-927 (1964). These disorders form the WAGR syndrome, and can be attributed to constitutional deletions of DNA in band 11p13 on human chromosome 11 in a group of genes known as the WAGR complex. Riccardi, et al., Pediatrics, 61:604-610 (1978); Francke, et al., Cytogenet. Cell Genet., 24:185-192 (1979). In these cases, bilateral Wilms' tumors are frequently observed, as are dysplastic changes in surrounding renal tissue (nephroblastomatosis) which are thought to precede malignant transformation. Bove and McAdams, Perspectives on Pediatric Pathol., 3:185-223 (1976). As a recessive oncogene or anti-oncogene, the Wilms' tumor locus curtails the growth of undifferentiated nephretic cells. It conforms generally to a two-mutation model of carcinogenesis and is genetically similar to the retinoblastoma locus on chromosome 13q. These observations lead to the conclusion that at least in this subset of Wilms' tumors, the inactivation of a gene in 11p13, analogous to the retinoblastoma (RB) gene, is a key event in tumor formation. Considerable effort has been expended in attempting to localize the gene responsible for WT, as is evidenced by the numerous reports describing such efforts. For example, genomic analysis of sporadic Wilms' tumors showing loss of heterozygosity at polymorphic loci supports the localization of Wilms' tumor gene to 11p13. Koufos, et al., Nature, 309:170-172 (1984); Orkin, et al., Nature, 309:172-174 (1984); Reeve, et al., Nature, 309:174-176 (1984); Fearon, et al., Nature, 309:176-178 (1984).

Based on additional research, it appears that Wilms' tumor may be caused by loss of function at alternative loci. In studies of two families showing hereditary predisposition to Wilms' tumor, linkage to 11p genetic markers was excluded, indicating the presence of at least one additional Wilms' tumor locus. Grundy, et al., Nature, 336:374-376 (1988); Huff, et al., Nature, 336:377-378 (1988). Further studies showed loss of heterozygosity in Wilms' tumors at 11pl5 rather than 11p13. Reeve, et al., Mol. Cell Biol., 9:1799-1803 (1989); Koufos, et al., Am. J. Hum. Gen., 44:711-719 (1989). Although these data suggest the possibility of additional loci, the 11p13 Wilms' tumor locus is clearly associated with constitutional WAGR deletions and somatic chromosome rearrangements in a subset of sporadic tumors. Lewis, et al., Genomics, 3:25-31 (1988).

Despite considerable interest in identifying the Wilms' tumor gene and work focusing on doing so, to the present time, a transcript mapping to the region identified as containing the Wilms' tumor gene has not been identified.

### Summary of the Invention

The present invention relates to the subject matter as set out in the claims. As used herein, the term Wilms' tumor gene or Wilms' tumor DNA refers to lesions in chromosome 11 band 13 (11p13) which are characteristic of WAGR or Wilms' tumor (i.e., found in cells affected in these conditions), but which can reasonably be expected to be associated with or causative of other tumor types. The present invention further relates to DNA sequences, both genomic and cDNA clones, which map within the boundaries of constitutional and tumor deletions which define the Wilms' tumor locus on human chromosome 11 band p13 (11p13). For the first time, a transcript which maps to the region containing the Wilms' tumor gene has been identified. The transcript has been characterized and shown to span approximately 50 kb and to encode an mRNA (referred to as WT mRNA) approximately 3 kb in length. The WT mRNA has been shown to be expressed in a limited number of cell types (i.e., predominantly kidney cells and a subset of hematopoietic cells).

The amino acid sequence of the polypeptide encoded by the sequence has also been derived and features of the polypeptide have been examined. Several of these features, such as the presence of four zinc finger domains and of a region rich in proline and glutamine, are indicative of a role in transcription regulation. The localization of the gene to 11p13, its tissue-specific expression and its predicted function, support the conclusion that it is the 11p13 Wilms' tumor gene. The present invention includes a method of identifying the Wilms' tumor gene; the isolated Wilms' tumor gene, the isolated gene transcript; the isolated encoded polypeptide; and diagnostic methods and reagents based thereon. The present invention makes available for the first time a method of identifying in a sample DNA which is clearly within the 11p13 Wilms' tumor locus, an mRNA transcript thereof or a Wilms' tumor-encoded polypeptide, as well as materials (e.g., nucleic acid probes, anti-Wilms' tumor polypeptide antibodies) useful in the method. This is particularly valuable because although Wilms' tumor is highly malignant and grows rapidly, it represents one of the clearest examples of success in pediatric oncology, as a result of the development of effective therapeutic regimens. The present invention provides a means by which the risk of developing WAGR or Wilms' tumor can be assessed prior to its appearance and the presence of the disease, once it has occurred, can be confirmed, thus making it possible to intervene therapeutically prior to or at an early stage in the development of the disease. It also provides a method by which the occurrence of DNA of the same or similar sequence as the Wilms' tumor gene can be detected in other tumor types (e.g., leukemia cells, testicular tumors), using DNA probes or antibodies specific for Wilms' tumor gene-encoded polypeptide.

### Brief Description of the Figures

Figure 1 is a schematic representation of isolation and characterization of the Wilms' tumor gene.

Figure 2 is a schematic representation of the WAGR region showing the map positions of single copy probes J7-18p2, J8-3p4 and cDNAs homologous to J8-3.

Figure 3 shows the nucleotide sequence of WT33 cDNA and the predicted amino acid sequence of the open reading frame extending from nucleotide 1 to 1035. The proline and glutamine residues in the proline/glutamine rich region (nucleotides 6 to 468) are boxed and the amino acids of the four zinc fingers (nucleotides 670 to 1002) which fit the zinc finger consensus are underlined.

Figure 4 is a schematic map of the WT33 cDNA; the open reading frame is shown in the boxed region and the deduced amino acid sequence of the proline/glutamine rich region appears above the shaded open reading frame.

Figure 5 is a schematic map showing a comparison of the sequence derived from WT33 to the zinc finger consensus region and the sequences of human EGR1 and EGR2 genes.

Figure 6 shows the results of Southern blot analyses of hybridization of Wilms' tumor cDNA with various DNA.

Figure 6A shows the results of Southern blot analysis of EcoRI digested human lymphoblast DNA hybridized with WT33.

Figure 6B shows the results of Southern blot analysis of Wit-13 hybrid cell lines digested with Eco RI and hybridized with WT2.

Figure 7 shows the genomic organization of six overlapping cosmids encoding the WT33 mRNA; a composite Eco RI restriction map of the 93 Kbp genomic region spanning the WT33 cDNA is shown at the top of the Figure.

Figure 8 shows the results of Northern blot analysis of the expression of WT33 in various tissues and tumor cell lines.

Figure 8A shows the results of Northern blot analysis of the tissue specific expression of WT33 in baboon.

Figure 8B shows the results of Northern blot analysis of the tissue specific distribution of RNA from mouse and baboon tissues.

Figure 8C shows the results of Northern blot analysis of expression of WT33 in tumor cell lines.

Figure 9 shows the expression of a Wilms' tumor fusion protein.

### Detailed Description of the Invention

The present invention is based on the identification and characterization of the Wilms' tumor gene and the mRNA transcript of the gene, as well as on the characterization of the polypeptide encoded by the Wilms' tumor gene. As described in detail below, a series of genomic and cDNA clones which map within the boundaries of constitutional and tumor deletions which define the Wilms' tumor locus on chromosome 11 band p13 (11p13) have been isolated and characterized. As also described below, the expression pattern of mRNA encoded by the transcription unit which corresponds to the clones has been determined. In addition, the polypeptide encoded by the Wilms' tumor locus has been characterized and shown to have several features which suggest it has a role in the regulation of transcription.

Based on the work described herein, a method of determining the presence or absence of Wilms' tumor DNA, as well as quantitating Wilms' tumor DNA in cells has been developed. Nucleic acid probes which hybridize to Wilms' tumor DNA and nucleic acid probes which hybridize to transcripts of the Wilms' tumor DNA have also been produced and used in the method. Although it is referred to herein as the Wilms' tumor gene or Wilms' tumor DNA, the locus on Chromosome 11 band 13 is referred to in this manner for convenience and is not meant to limit the present invention to the medical conditions designated WAGR and Wilms' tumor only. Thus, it is reasonable to expect that the 11p13 locus referred to as Wilms' tumor DNA occurs in (is associated with or causative of) other tumor types, such as leukemia cells and testicular tumors. The present invention is intended to include such an occurrence and provides a method by which the equivalent gene or DNA sequence (i.e., a DNA sequence which cross hybridizes with a probe as described herein and acts as a recessive oncogene or anti-oncogene in cells in which it occurs) can be identified in other types of tumors.

The following is a description of isolation and characterization of Wilms' tumor genomic DNA and cDNA, the mRNA transcript and the encoded polypeptide.

Molecular mapping experiments have narrowed the WAGR regions to a specific interval in 11p13 bounded by the genes encoding erythrocyte catalase (CAT) and the subunit of follicle stimulating hormone (FSHB). Junien et al., Am. Genet., 23:16-168 (1980); van Heyningen, et al., Proc. Natl. Acad. Sci.. USA, 82: 8592-8596 (1985); Glaser, et al., Nature, 321:882-887 (1986); Porteous, et al., Proc. Natl. Acad. Sci. USA, 84:5355-5359 (1987); Watkins, et al., DNA, 6:205-212 (1987). Three complementary strategies have been used to further delineate the location of genes within the WAGR region: somatic cell genetics, molecular cloning and pulsed field gel electrophoresis. Somatic cell hybrids segregating specific translocation and deletion chromosomes have been valuable reagents for resolving and defining the position of individual genes within the WAGR region. A substantial number of additional 11p13 DNA markers have been isolated and characterized from chromosome 11-specific DNA libraries. Lewis, et al, ibid (1988); Compton, et al., Cell, 55:827-836 (1988); Davis, et al., Genomics, 3:24-27 (1988a); Davis, et al., Science, 241:840-842 (1988b); Gessler, et al., J. Am. Hum. Genet., 44:486-495 (1989a); Gessler, et al., Science, 244:1575-1572 (1989b). Long range restriction maps constructed by pulsed field gel electrophoresis define relatively large intervals for several of the WAGR disease genes.

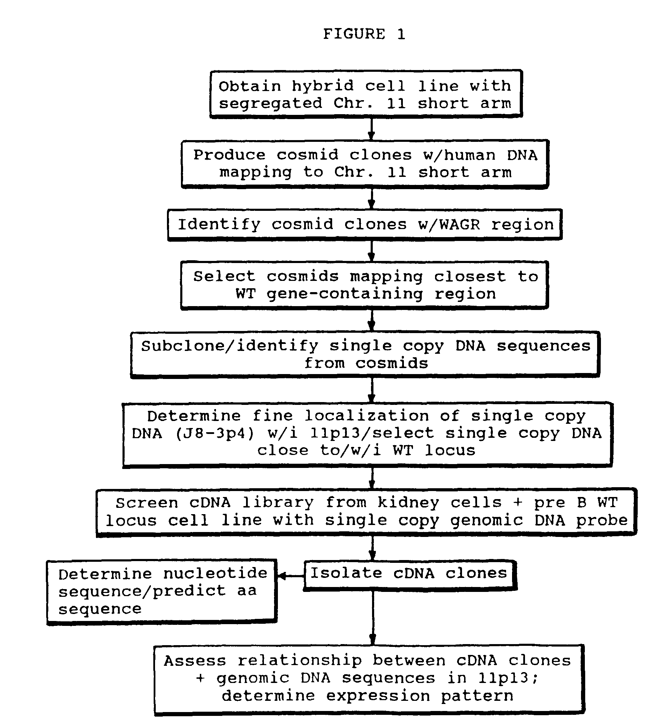

The method by which Wilms' tumor DNA was isolated is represented schematically in Figure 1. Initially, a hamster-somatic human cell line (J1-11), in which the short arm of human chromosome 11 had been segregated from the remainder of the human genome, was used to produce cosmid libraries, as described in the Exemplification. One hundred nineteen cosmid clones, all containing human DNA which mapped to the short arm of chromosome 11, were isolated from the library. Clones containing the WAGR region were subsequently identified, using a mapping panel of somatic cell hybrids containing different fragments of human chromosome 11p. Of the clones isolated in this manner, three (J7-18, J8-3 and J10-15) appeared to map most closely to the region containing the Wilms' tumor gene. The restriction maps of J8-3 and J10-15 showed substantial overlap and, therefore, only one of these cosmids (J8-3) was analyzed further.

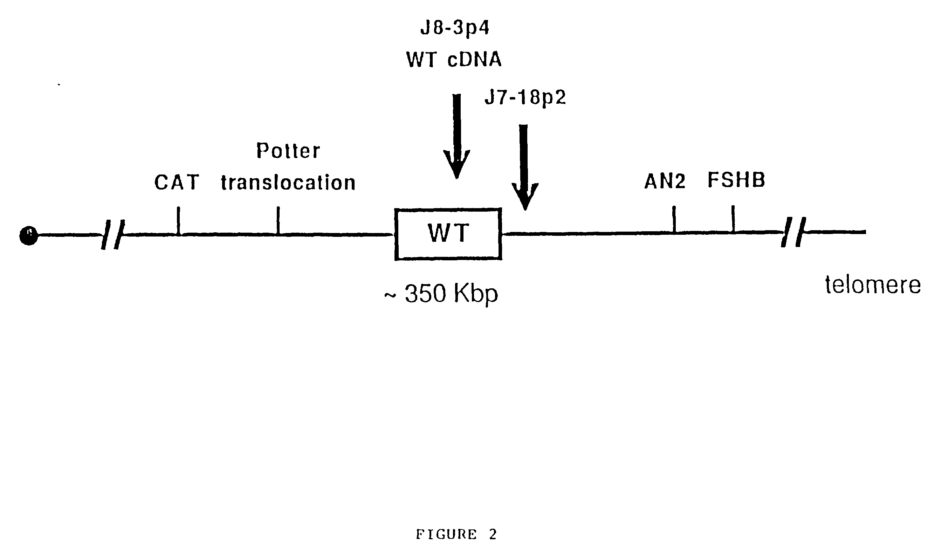

Single copy sequences, designated J7-18p2 and J8-3p4, were subcloned and identified from cosmids J7-18 and J8-3, respectively. The fine localization of these single copy DNA sequences was determined by hybridization to a series of somatic cell hybrids derived from patients with translocations and deletions which define specific intervals within the WAGR region. This is described in detail in the Exemplification and a map summarizing the findings is shown in Figure 2.

J8-3p4 was used as a probe to screen cDNA libraries. J8-3p4 was selected for this purpose because its map position indicated that it was close to or within the Wilms' tumor locus. In addition, as explained in the Exemplification, two observations suggested that J8-3p4 contained a portion of a transcription unit. A cDNA library derived from human embryonic kidney (HEK) cells was screened with J8-3p4. On the basis of Northern blotting results (see the Exemplification), a human adult kidney library and a human pre B cell library were also screened. Four cDNA clones from these three libraries were studied in detail: two from HEK (WT4, WT2), one from human adult kidney (WT22) and one from a pre B cell line (WT33). Another homologous cDNA clone (WT13) was isolated from the HEK library, using an independently isolated conserved genomic DNA clone, λK13. Glaser, T., The fine structure and evolution of the eleventh human chromosome. Ph.D. thesis, Massachusetts Institute of Technology, Cambridge, MA (1988).

cDNA clone WT33 is 2313 base pairs (bp) in length and the longest clone isolated. It extends the furthest in both the 5' and the 3' directions of the clones isolated. The other four cDNAs share a common internal region of DNA sequence approximately 1000 to 1200 bp in length.

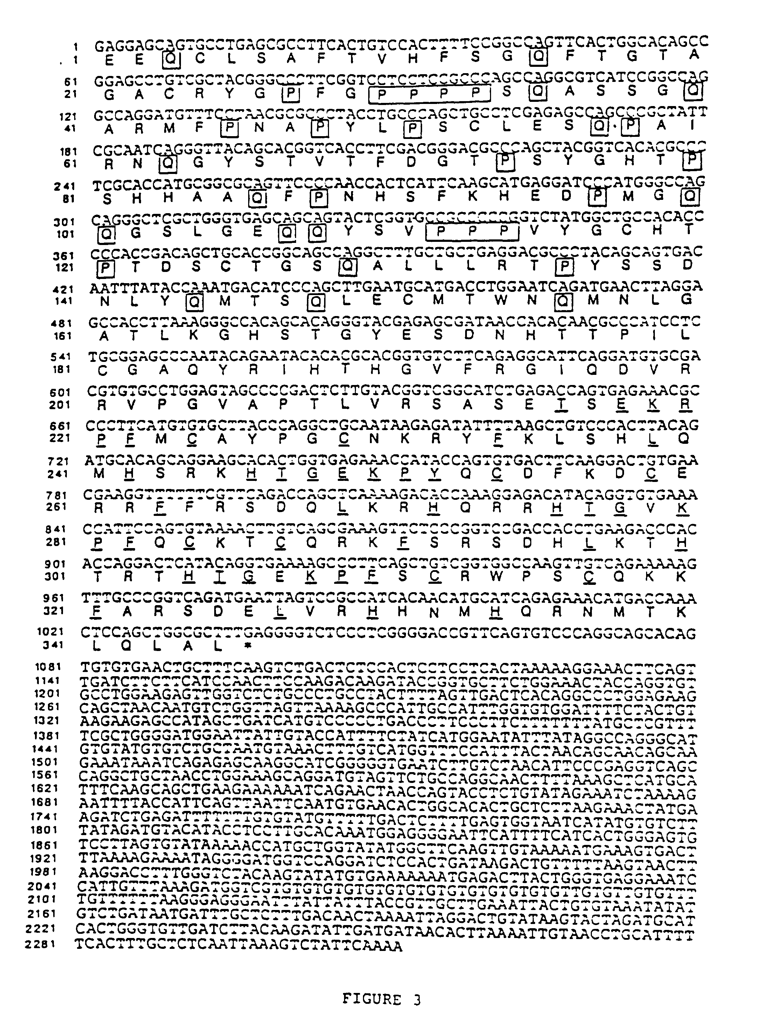

cDNA clone WT33 was selected for further analysis, which is described in detail in the Exemplification. The WT33 nucleotide sequence was determined and the predicted amino acid sequence was derived. Both are represented in Figure 3. Sequence analysis showed the presence of a continuous open reading frame of 345 amino acids, which extends from nucleotides 1 to 1035. This open reading frame appears to represent most of the WT33 coding segment, but it does not appear to include the initiator methonine codon. Primer extension experiments suggest that an additional 200 bp are present at the 5' end of the mRNA corresponding to WT33. The transcription pattern of the locus corresponding to these cDNAs exhibits some complexity. Experiments utilizing RNA PCR (polymerase chain reaction) indicate variation in mRNA sequence in the 5' segment of the coding region of the mRNA, suggesting alternative splicing patterns among various tissue types.

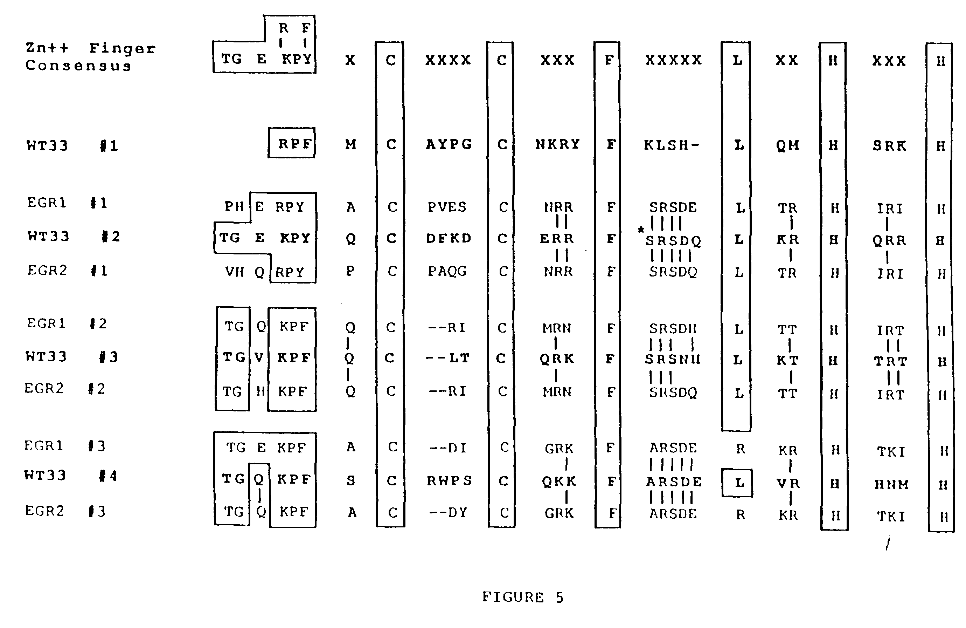

Of particular interest is that nucleotides 670 to 1002 encode four contiguous "zinc finger" domains. All four zinc fingers encoded by WT33 (Figure 5) fit the consensus sequence for zinc fingers (Miller, J., et al. EMBO J., 4:1609-1614 (1985); Evans, R.N., et al., Cell, 52:1-3 (1988). The H/C link between zinc fingers, typified by the amino acid sequence TGE-R/K-P-F/Y-X, is also conserved in the deduced amino acid sequence. Shuh, R., et al., Cell, 47:1025-1032 (1986).

A search of other polypeptides for sequences related to WT33 revealed a 51% similarity between the amino acid sequence of the zinc finger region of two recently identified human early growth response genes, EGR1, Sukhatme, et al., Cell, 53:37-43 (1988) and EGR2, Joseph, et al., Proc. Natl. Acad. Sci. USA, 85:7164-7168 (1988). The early growth response genes have been suggested to be involved in pathways controlling cell proliferation. The individual zinc fingers of WT33 are aligned with the zinc finger consensus sequence and compared with the zinc fingers of EGR1 and EGR2 in Figure 5. Although the WT33 polypeptide has homology to zinc fingers in other proteins, including TFIIIA and Spl, the degree of homology is greatest with EGR1 and EGR2 and moreover was observed throughout all three contiguous zinc fingers.

The amino acid content of the region 5' amino terminal to the zinc finger domain is also characteristic of proteins thought to be transcription factors. From the amino terminus to the start of the first zinc finger, there is a high concentration of serine (10.2%), proline (9.8%), glycine (9.7%), threonine (8.8%) and glutamine (7.9%) residues. These amino acids are also highly represented in the amino termini of the polypeptides encoded by EGR1 and EGR2. Proline and glutamine rich domains have been identified as motifs in a number of transcription factors and putative transcription factors. Mitchell and Tjian, Science, 245:371-378 (1989). A high threonine and serine content is also observed in several transcription factors, including Spl. Courey, et al., Cell, 55:887-898 (1988).

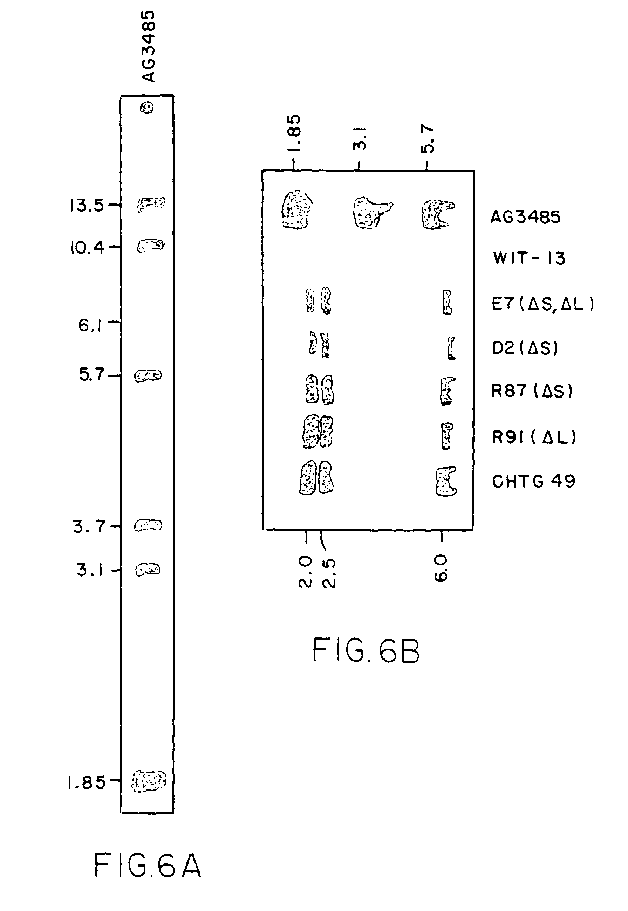

The relationship between cDNA clones isolated as described and genomic DNA sequences in 11p13 was also addressed, as described in detail in the Exemplification. Briefly, segments of the WT33 cDNA were hybridized to genomic DNA from diploid human cell lines and to a panel of somatic cell hybrids which permits fine structure mapping within 11p13 (Table). As shown in Figure 6A, WT33 cDNA hybridizes to seven EcoRI fragments in normal human DNA which are 13.5, 10.4, 6.1, 5.7, 3.7, 3.1, and 1.85 kb in length. Analysis of somatic cell hybrids confirmed that all of these restriction fragments are located on chromosome 11 in band p13. Furthermore, these DNA sequences are all homozygously deleted from cell line WiT-13 and hybrids derived from this line. (Figure 6B and data not shown).

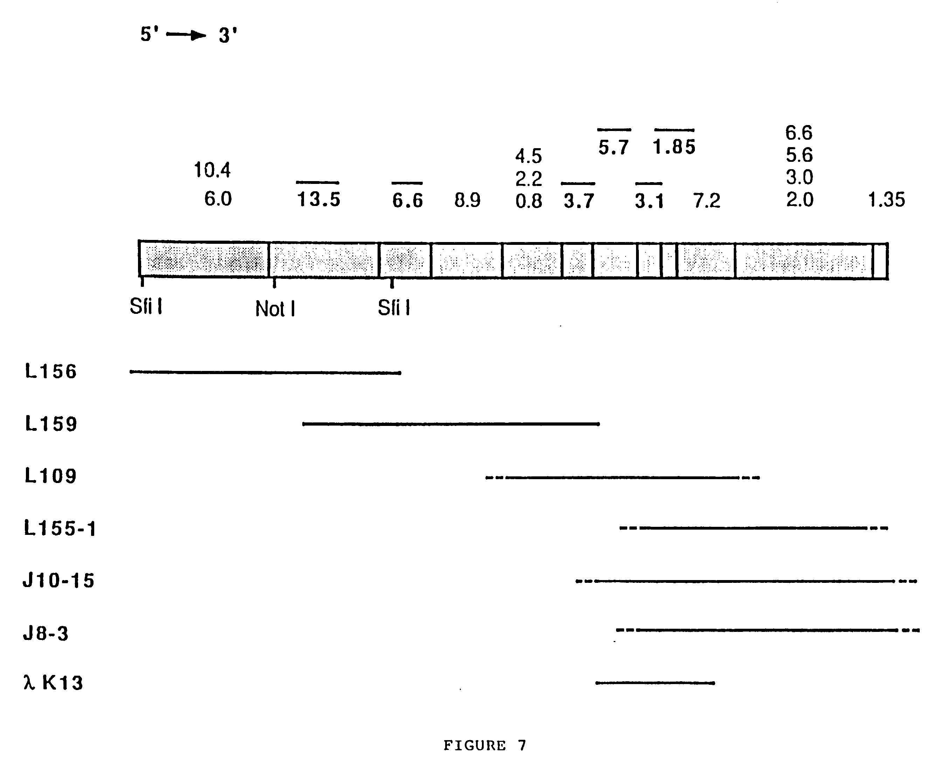

To further analyze the structure of the genomic DNA within the region, WT33 was used as a probe to isolate additional cosmid DNA clones. Figure 7 shows a composite map of four cosmids derived from this analysis (L156, L159, L109, L155-1) plus the two original cosmids, J8-3 and J10-15, and phage clone λK13. Glaser, T., The fine structure and evolution of the eleventh human chromosome. Ph.D. thesis, Massachusetts Institute of Technology, Cambridge, MA (1988). The cloned genomic sequences span a DNA segment greater than 90 kb in length. To relate genomic and cDNA clones, an Eco RI digest of each cosmid was hybridized with segments of WT33 cDNA. In this manner, all seven Eco RI fragments observed by Southern hybridization of the cDNA to genomic DNA were identified within this collection of overlapping clones (Figure 7). Orientation of the transcriptional unit was established by hybridizing restriction digests of each cosmid with probes derived from different subregions of the WT33 cDNA. (See the Exemplification). These data indicate that the WT33 transcriptional unit must extend from a position close to the Not I site in cosmid L156 and continue in the 3' direction, extending through the 1.85 kb Eco RI fragment common to cosmids L109, L155-1, J10-15, J8-3 and clone λK13. These hybridizing Eco RI fragments span approximately 50 kb. Since WT33 cDNA is not full length (See above), the entire gene may be greater in size than 50 kb.

An analysis of restriction enzyme recognition sites in cloned genomic DNA permits a direct comparison to the pulsed field gel electrophoresis map of the region. As shown in Figure 7, the 5' end of the genomic DNA segment encoding the 5' end of WT33 cDNA includes a recognition site for the restriction enzyme Not I. Pulsed field gel mapping demonstrates that the 11p13 Wilms' tumor gene is located within the boundaries of two adjacent Not I fragments, 500 kb and 325 kb in length. Hybridization to genomic DNA digested with both Sfi I and Not I confirms that the Not I site in cosmid L156 represents the junction between the 325 kb and 500 kb Not I restriction fragments. Since pulsed field gel analysis places the 500 kb Not I fragment centromeric to the 325 kb Not I fragment, transcription must proceed in a centromeric to telomeric direction.

Cosmid L156 contains sites for a number of restriction enzymes with recognition sequences which contain the dinucleotide CpG, including Not I, BssH II and Eag I. These data, as well as pulsed field gel analysis, indicate the presence of an "HTF island" in the region of genomic DNA surrounding the Not I site. HTF islands are frequently located at the 5' ends of transcription units, Bird, A., et al., Cell, 40:91-99 (1985); Bird, A., Nature, 321:209-213 (1986); Lindsay, S, et al., Nature, 327:336 (1987), suggesting that the genomic DNA in cosmid L156 may contain the 5' end of the WT33 transcription unit.

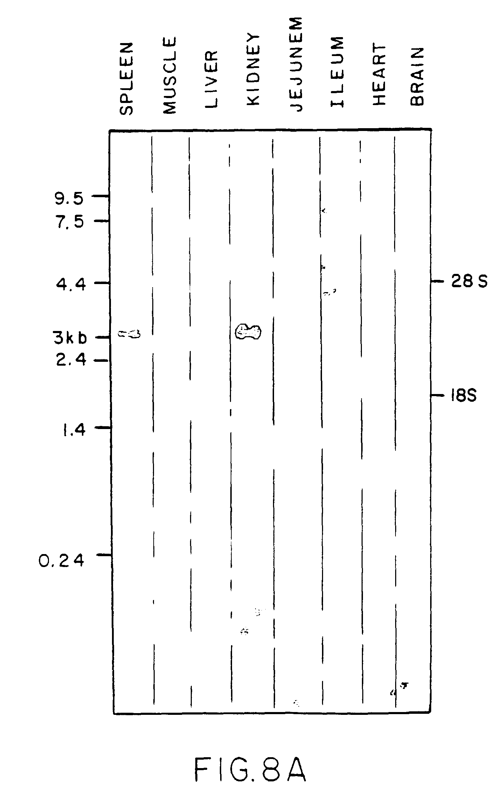

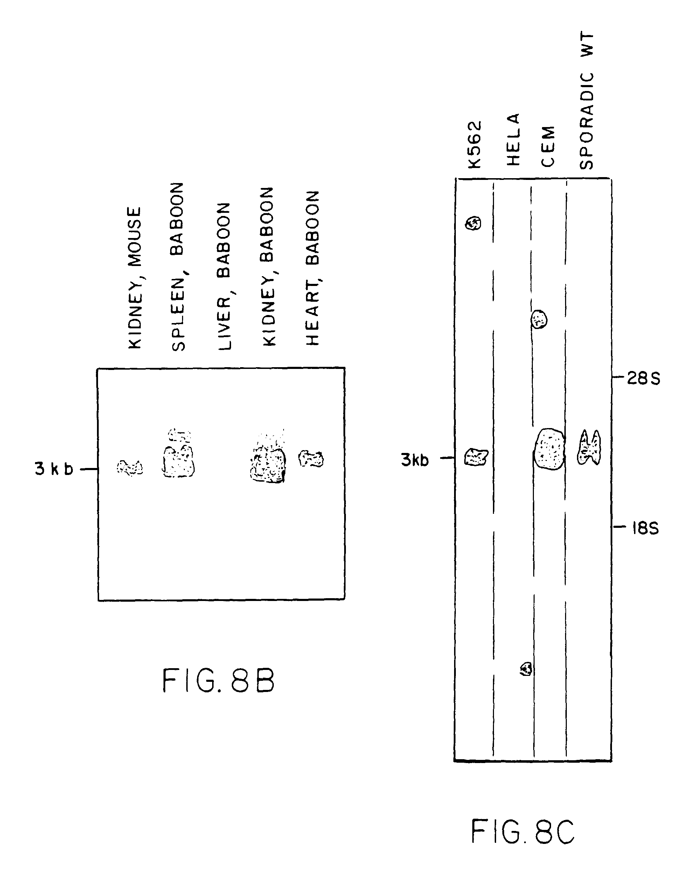

The size and tissue distribution of the WT33 transcript(2), were also assessed, by performing a series of Northern blotting experiments. Figure 8A shows the hybridization of WT33 cDNA to total cellular RNA isolated from a variety of baboon tissues. A mRNA species approximately 3kb in length is detected in baboon kidney and spleen RNA. A faint hybridization band at 3 kb is also observed in heart upon long exposure (Figure 8B), while no detectable hybridization is observed in RNA derived from muscle, liver, jejunum, ileum or brain (Figure 8A). WT33 is an effective probe in hybridization to RNA derived from mouse tissues as well. A 3 kb mRNA species homologous to WT33 is observed in mouse tissues as well. A 3 kb mRNA species homologous to WT33 is observed in mouse kidney (Figure 8B). The tissue specific expression pattern of WT33 mRNA in the adult mouse is similar to the baboon. Developmental studies in the mouse show that the WT33 mRNA is most highly expressed in fetal kidney. This expression is consistent with a gene capable of growth regulation in the metanephric blastema, the presumed tissue of origin for Wilms' tumor. Bove, et al., (1976) ibid. The finding of homology with the EGR1 and EGR2 genes also suggests WT33 may exert a role in the growth regulation of nephroblasts.

A spectrum of tumor cell lines, including two neuroblastomas (SK-N-Be(2) and NGP), a retinoblastoma (WERI) a breast carcinoma (MCF7), two osteosarcomas (HOS and U205), two melanomas (SK-MEL-130 and SK-MEL-147), a bladder carcinoma (Ej), two colon carcinomas (SE480 and WIDR), a cervical carcinoma (HeLa) and two Esptein-Barr virus transformed B cell lines (TSH-1 and TSH-2) did not show detectable hybridization to WT33 cDNA. In contrast, RNA isolated from several sporadic Wilms' tumors showed strong hybridization to WT33 cDNA at the 3 kb position. An example is shown in Figure 8C. Similarly, RNA isolated from two hematopoietic cell lines, an erythroleukemia (K562) and an acute lymphocytic leukemia (CEM), also showed strong hybridization to WT33 at the 3 kb position (Figure 8C).

Results demonstrated expression of the WT33 transcript in cells of kidney and a subset of hematopoietic cell lines. These results are consistent with the tissue-specific expression observed predominantly in the baboon kidney and spleen.

Thus, using the method described, DNA which corresponds to the Wilms' tumor gene was identified, isolated and sequenced. The DNA has been shown to encode a transcription unit which spans approximately 50 kb and encodes an mRNA approximately 3 kb in length. This mRNA is expressed in a limited range of cell types, predominantly in the kidney and a subset of hematopoietic cells. The polypeptide encoded by this locus has a number of features which suggest a potential role in the regulation of transcription. These include the presence of four zinc finger domains and a region rich in proline and glutamine. The amino acid sequence of the predicted polypeptide shows significant homology to two growth regulated mammalian polypeptides EGR1 and EGR2. The genetic localization of this gene, its tissue-specific expression, and the function predicted from its sequence indicate that it represents the 11p13 Wilms' tumor gene.

As a result of the isolation and characterization of the Wilms' tumor gene, a method by which samples can be analyzed for the Wilms' tumor gene or a representative portion of the gene is available, as are reagents (e.g., nucleic acid probes, antibodies) useful in the method. This method can be used for diagnostic purposes, such as in assessing the likelihood/risk of development of WAGR syndrome and/or Wilms' tumors and in determining in an individual who presents with symptoms associated with or possibly indicative of WAGR or Wilms' tumor whether the disease is present or not. For example, cells obtained from an individual can be probed with all or a portion of the nucleotide sequence represented in Figure 3, using known techniques. The nucleotide sequence of such a probe need not be precisely the same as that in Figure 3. It need be only sufficiently similar to the sequence of Figure 3 that it will hybridize to the Wilms' tumor gene under the conditions used. Cells (e.g., blood, kidney) can be obtained prenatally or postnatally and the occurrence of the Wilms' tumor gene assessed. Cells can be analyzed for the Wilms' tumor DNA, the encoded RNA transcript and/or polypeptides encoded by the Wilms' tumor gene. For example, cells can be obtained, prenatally or postnatally, and analyzed for Wilms' tumor DNA. This can be carried out using standard blotting techniques, (e.g., Southern blot) and a radioactively labelled DNA probe which hybridizes to (is complementary to) all or a portion of the Wilms' tumor. A radioactively-labelled DNA probe can be combined with cellular DNA previously treated to render it available for hybridization with complementary DNA, under conditions appropriate for hybridization to occur. After sufficient time for the labelled DNA probe and complementary DNA in the sample (if present) to hybridize and form labelled DNA probe/sample DNA complexes, detection of the labelled probe/sample DNA complexes is carried out using known methods (e.g., autoradiography). The label can be any substance which can be detected and whose presence does not interfere with the availability to probe DNA to bind to complementary DNA (e.g., fluorescent material). The method by which labelled DNA probe/sample DNA complexes are detected will depend on the type of label used (e.g., in the case in which a fluorophore is used, fluorescence detection will be used). If necessary, DNA obtained from the sample can be amplified, using a known technique such as the polymerase chain reaction, and the amplified DNA analyzed for the occurrence of Wilms' tumor DNA. If sample DNA is amplified, the product is an amplification mixture which contains amplified DNA of interest (DNA which includes Wilms' tumor DNA) and amplified DNA other than DNA of interest. Generally, DNA in the amplification mixture is separated on the basis of size, using known techniques. Chemical detection methods can also be used. The separated amplified DNA is analyzed for DNA of interest using a known technique, such as Southern blotting, DNA sequencing, digestion with appropriate restriction endonuclease or visualization of ethidium bromide stained gels.

Alternatively, mRNA can be detected in the sample obtained, using as a probe all or a portion of the Wilms' tumor gene. This can be carried out using mRNA obtained from an individual's cells, or using mRNA obtained from cells and amplified using a known amplification technique, such as the RNA PCR. In either case, RNA is analyzed using a known technique, such as Northern blotting.

Antibodies specific for the Wilms' tumor gene-encoded polypeptide (or a polypeptide portion) can also be used for diagnostic purposes. Such antibodies can be produced using known techniques. Maniatis, T., et al., Molecular Cloning: A Laboratory Manual, Cold Spring Harbor (1982). In this embodiment, antibodies specific for the polypeptide encoded by the Wilms' tumor gene (e.g., as encoded by all or a portion of the sequence of Figure 3 or its functional equivalent) are combined with a sample (e.g., kidney cells, blood cells) obtained from an individual. The antibody used can be detectably labelled (e.g., with a radioactive or fluorescent material). After sufficient time for polypeptide present in the sample and antibody to combine or bind, to form Wilms' tumor gene-encoded polypeptide/specific

antibody complexes, the occurrence (presence or absence and/or quantity) of complexes is determined, using known techniques. If labelled specific antibody is used, the occurrence of labelled complexes is determined (e.g., by autoradiography, fluorescence detection). Alternatively, the sample can be combined with a solid support (e.g., nitrocellulose, glass slide, polystyrene beads, immunomagnetic beads) which bears an antibody specific for the antibody present in the complex. This results in binding of specific antibody in the sample (e.g., in the polypeptide/specific antibody complexes) to the solid support. The resulting solid support-bound complex can be removed from the sample and detected using known techniques. For example, if the antibody in the Wilms' tumor gene-encoded polypeptide/specific antibody complex is labelled, detection of the support-bound complex is carried out by detecting the label.

The above probes (e.g. DNA probes, mRNA probes and antibody probes) are used to determine differences in the Wilms' Tumor gene in individuals. These differences can be the aforementioned deletions or substitutions in the gene. One preferred portion of the gene to look at is that encoding the zinc finger regions or their expression product, preferably the nucleotides coding for at least one of the zinc finger regions; the antibody probe detects affinity with the residue itself. As aforesaid, the probes are to the entire gene shown in Figure 3 or portions thereof. As known in the art, such portions should be at least about 20 nucleotides in length. In one preferred embodiment, a probe serves as at least one of the primers for a PCR to determine deletions or alterations in the gene by looking at the DNA or mRNA, as known in the art based upon the present disclosure. The detection of such a deletion or alteration of a functional portion is indicative of a cancerous or precancerous condition. These probes can look at the cell or fluid from a human. For example, taking a predetermined cell or fluid sample from a human, adding the probe to the cell or fluid sample and determining from the probe whether there has been an alteration or deletion of a function portion of the Wilms' tumor gene, wherein such an alteration or deletion is indicative of the cancerous or precancerous condition. Standard techniques such as Southern blot, Northern blot, PCR, ELISA, immunoassay can be used to make the determination.

The present method is useful for early detection of WAGR and Wilms' tumor and, as a result, earlier intervention, in the form of surgery, chemotherapy and/or radiation therapy, will be possible. For example, the present method can be used to diagnose this condition in a patient who has an enlarging abdominal mass, abdominal pain, hematuria or constitutional symptoms (e.g., fever, vomiting, poor appetite, malaise, polycythemia, hypertension) suggestive of Wilms' tumor. That individual can, after diagnosis through use of the present method, be treated as described.

The present method of detecting the Wilms' tumor gene can also be used to identify in other tumor types a lesion which is the same as or similar to the lesions which occur in the case of Wilms' tumor. That is, it is reasonable to expect that the Wilms' tumor gene (i.e., the DNA sequence referred to herein as the Wilms' tumor gene) or a closely-related gene is expressed in other tumor types (e.g., leukemia cells, testicular tumor) and that it is casually associated with those tumor types or serves as a reliable indicator (marker) of such tumor types, although perhaps not directly or solely responsible for formation of a particular type of tumor. Thus, the present method and appropriate reagents, such as DNA sequences within the cosmid clones described herein or the Wilms' tumor gene itself, can be used to identify in other tumor types similar lesions in chromosome 11 band 13. The DNA sequences described herein can be used to identify in a tumor sample (e.g., leukemia cells, testicular tumor) an altered 11p13 sequence, using known techniques and the method described herein.

The polypeptide or an immunogenic portion thereof can be used for therapeutic or diagnostic purposes.

For example, a vector containing a Wilms' tumor gene cDNA sequence, preferably, the sequence shown in Figure 3 or the functional equivalent thereof, containing an ATG initiation sequence, polyadenylation sequences downstream 3' from the cDNA sequence and preferably also containing a replication origin can be used to transfect a cell. The vector will also contain a promoter to permit expression of the suppressor gene. Promoters used in the vector can be any of the known promoters and the choice is governed by the host cell one wishes to use to thereby permit expression of the desired product in the host cell of choice. Retroviral promoters are preferred. Such promoters include retroviral promoters such as Akv, SL3-3 and Friend viruses. The vector can preferably also contain an enhancer, such enhancers are well known in the art, for example, viral enhancer. More preferably, it contains an enhancer which is tissue specific. Preferably, the vector also contains a marker gene to aid in detection of transformed cells. Such markers are well known in the art and can include an antibiotic resistance gene such as the bacterial neomycin resistance gene, which confers a dominant selectable resistance to the antibiotic G418 in eukaryotic cells.

Although different cell lines can differ in their ability to take up and express the transfected suppressor gene DNA by use of appropriate promoters, a wide range of host cells can be used. For example, NIH3T3, CHO, COS, SF-9(ATCC No. CRL 1711) and SF-21 are preferred. Typically, mammalian cells would be preferred, but the cell lines of the present invention are not limited thereto. For example, bacterial cells such as E. coli can be used (see Exemplification).

The vector can be used to transform cell lines, for example, by being introduced into psi/2 (ecotropic) and psiAM (amphotropic) cell lines by a variety of methods well known to the art. The preferred method is the calcium phosphate co-precipitation method See Wigler, et al, Cell 16:777-785 (1979). These cell lines can constitutively produce infectious, replication defective murine leukemia viruses containing a genome derived from the vector. Cone, et al, PNAS 81:6349-6353 (1984); Mann, et al, Cell 33:153-159 (1983). Two days following transfection, cells can be selected by looking for the marker, i.e. antibiotic resistance gene, which the vector would also preferably contain. Antibiotic resistant clones, e.g. G418, would be evident in a short period of time, typically 7-10 days.

The antibiotic resistant psi/2 and psi/AM clones are isolated and clones producing large amounts of the suppressor gene product are selected. These cells are then cultured in a standard medium and harvested as needed.

The protein produced from these cells can then be used to generate an antibody to the suppressor gene product (protein). The antibody generated can be polyclonal or monoclonal depending upon the particular application for which it is designed. Such antibodies can be prepared by techniques well know to the skilled artisan. For example, the protein or an antigenic portion thereof can be conjugated to keyhole limpet hemocyanin (KLH) and used to raise an antibody in an animal such as a rabbit. Typically, the peptide-KLH conjugate is injected several times over a period of about two months to generate antibodies. The antibody is then collected from serum by standard techniques. Alternatively, monoclonal antibodies can be produced in cells which produce antibodies to the proteins by using standard fusion techniques for forming hybridoma cells. [Kohler, G., et al., Nature 256:495 (1975)]. Typically, this involves fusing an antibody producing cell with an immortal cell line such as a myeloma cell to produce the hybrid cell. Alternatively, monoclonal antibodies can be produced from cells by the method of Huse, et al, Science 246:1275 (1989).

For example, hybridomas can be generated by immunization of mice with viable cells transformed by the suppressor gene-containing vector, which express the suppressor gene product. For example, using the full length protein as the immunogen, it is possible to generate a collection of monoclonal antibodies with specificities that span the entire length of the protein. Preferably, when creating antibodies as probes, one will use the immunogenic portion of the polypeptide most likely to be probative of a defective protein and gene sequence such as any of the transcription regions, for example, any of the zinc finger regions. Preferably, one generates an antibody that will detect a deletion or alteration of any of the cysteine residues in these regions. The immunogenic peptides should be at least about 8 amino acids in length. The Wilms' tumor fusion protein shown in the Examplification can be used to generate antibodies by standard methods.

The mice can be immunized intraperitoneally (I.P.) with a sufficient number of viable cells of the host cell, which is to be transfected by the suppressor gene vector. This can then be followed immediately by an I.P. injection of, for example, cyclophosphamide in H₂O. The cyclophosphamide treatment is repeated one and two days following the primary injection. About two weeks following immunization, mice are then injected with a sufficient amount of the transformed host cells and then allowed to rest for another two weeks, at which time the entire procedure is repeated. Four days following the second injection of the transformed cells, the animals are sacrificed and their spleens obtained for the first fusion.

Hybridomas are produced by fusing cells by typical techniques, such as from immunized mice with SP2/O myeloma cells by a polyethylene glycol (PEG) method. Cells are asceptically removed from immunized mice and a single cell suspension of the spleen cells obtained by perfusing the spleen with serum-free media (e.g. DME). Spleen cells and myeloma cells are mixed together at a ratio, for example, 5 to 1, spleen cells to myeloma cells. The cells are then centrifuged and the supernatant removed by aspiration. The cells are then grown in medium by standard techniques. Hybridomas, which grow after the fusion procedure, are then screened for secretion of anti-human suppressor product antibodies by an ELISA assay on a cell lysate. Hybridomas, that produce positive results, are expanded and cloned by limiting dilution to assure that the cells and resulting antibodies are indeed, monoclonal. Hybridoma colonies, that test positive for the presence of antibody to the human suppressor gene product are diluted in media to a concentration of, for example, 5 hybridoma cells per mililiter. Once colonies grow, the supernatants are again tested for the presence of antibody to the human suppressor gene product. If the results are positive when tested by an ELISA assay, the colonies are cloned again by limiting dilution.

The resultant antibodies can then be used as probes in the assay method discussed above. Such antibody probes are particularly sensitive to alterations or deletions in a suppressor gene, which affect the resultant protein products. Thus, the antibody probes provide a simple and efficient means of determining whether a deletion or alteration of the gene has affected a functional unit. By using antibody probes it is possible to determine both the level of expressed protein and whether there has been a change in expression. One can compare results against base line levels obtained for the material being sampled, e.g., level of suppressor gene product in blood, or by comparing a test cell (preselected cell) from an individual with another cell from that individual, which is not believed to show malignancy. If there is a change (e.g., absence of reactive protein of altered electrophoretic mobility) it is indicative that the test cell is malignant. Further, one can take cell samples from the same individual at various times to provide levels of comparison. This can also be done for the nucleotide probes.

In accordance with this invention, an antibody or cocktail of probes, e.g. antibody probes, can be used for detection. The probes, e.g. antibodies, can be labelled directly with a reporter or indirectly with a member of a specific binding pair using conventional techniques.

Specific binding pairs can be of the immune or non-immune type. Immune specific binding pairs are exemplified by antigen-antibody systems of hapten/anti-hapten systems. These include fluorescein/anti-fluorescein, dinitrophenyl/anti-dinitrophenyl, biotin/anti-biotin, peptide/anti-peptide and the like. The antibody member of the specific binding pair can be produced by customary methods familiar to those skilled in the art. Such methods involve immunizing an animal with the antigen member of the specific binding pair. If the antigen member of the specific binding pair is not immunogenic, e.g., a hapten, it can be covalently coupled to a carrier protein to render it immunogenic.

Non-immune binding pairs include systems wherein the two components share a natural affinity for each other but are not antibodies. Exemplary non-immune pairs are biotin-streptavidin, intrinsic factor-vitamin B₁₂, folic acid-folate binding protein and the like.

A variety of methods are available to covalently label antibodies with members of specific binding pairs. Methods are selected based upon the nature of the member of the specific binding pair, the type of linkage desired, and the tolerance of the antibody to various conjugation chemistries. Biotin can be covalently coupled to antibodies by utilizing commercially available active derivatives. Some of these are biotin-N-hydroxy-succinimide which binds to amine groups on proteins; bitoin hydrazide which binds to carbohydrate moieties, aldehydes and carboxyl groups via a carbodiimide coupling; and biotin maleimide and iodoacetyl biotin which bind to sulfhydryl groups. Fluorescein can be coupled to protein amine groups using fluorescein isothiocyanate. Dinitrophenyl groups can be coupled to protein amine groups using 2,4-dinitrobenzene sulfate or 2,4-dinitrofluorobenzene. Other standard methods of conjugation can be employed to couple monoclonal antibodies to a member of a specific binding pair including dialdehyde, carbodiimide coupling, homofunctional crosslinking, and heterobifunctional crosslinking. Carbodiimide coupling is an effective method of coupling carboxyl groups on one substance to amine groups on another. Carbodiimide coupling is facilitated by using the commercially available reagent 1-ethyl-3-(dimethyl-aminopropyl)-carbodiimide (EDAC).

Homobifunctional crosslinkers, including the bifunctional imidoesters and bifunctional N-hydroxy-succinimide esters, are commercially available and are employed for coupling amine groups on one substance to amine groups on another. Heterobifunctional crosslinkers are reagents which possess different functional groups. The most common commercially available heterobifunctional crosslinkers have an amine reactive N-hydroxysuccinimide ester as one functional group, and a sulfdhydryl reactive group as the second functional group. The most common sulfhydryl reactive groups are maleimides, pyridyl disulfides and active halogens. One of the functional groups can be a photoactive aryl nitrene, which upon irradiation reacts with a variety of groups.

The detectably-labelled probe, e.g., antibody, detectably-labelled antibodies, or detectably-labelled member of the specific binding pair is coupled to a reporter which can be a radioactive isotope, enzyme, fluorogenic, chemiluminescent or electrochemical materials. Two commonly used radioactive isotopes are ¹²⁵I and ³H. Standard radioactive isotopic labeling procedures include the chloramine T, lactoperoxidase and Bolton-Hunter methods for ¹²⁵I and reduction methylation for ³H.

Enzymes suitable for use in this invention include, but are not limited to, horseradish peroxidase, alkaline phosphatase, β-galactosidase, glucose oxidase, luciferase, β-lactamase, urease and lysozyme. Enzyme labeling is facilitated by using dialdehyde, carbodiimide coupling, homobifunctional crosslinkers and heterobifunctional crosslinkers as described above for coupling an antibody with a member of a specific binding pair.

The labeling method chosen depends on the functional groups available on the enzyme and the material to be labeled, and the tolerance of both to the conjugation conditions. The labeling method used in the present invention can be one of, but not limited to, any conventional methods currently employed including those described by Engvall and Pearlmann, Immunochemistry 8:871 (1971), Avrameas and Ternynck, Immunochemistry 8:1175 (1975), Ishikawa et al., J. Immunoassay 4 (3):209-327 (1983) and Jablonski, Anal. Biochem. 148:199 (1985).

Labeling can be accomplished by indirect methods such as using spacers or other members of specific binding pairs. An example of this is the detection of a biotinylated antibody with unlabelled streptavidin and biotinylated enzyme, with streptavidin and biotinylated enzyme being added either sequentially or simultaneously. Thus, according to the present invention, the antibody used to detect can be detectably-labelled directly with a reporter or indirectly with a first member of a specific binding pair. When the antibody is coupled to a first member of a specific binding pair, then detection is effected by reacting the antibody-first member of a specific binding complex with the second member of the binding pair which is labelled or unlabelled as mentioned above.

Moreover, the unlabelled detector antibody can be detected by reacting the unlabelled antibody with a labelled antibody specific for the unlabelled antibody. Such an anti-antibody can be labelled directly or indirectly using any of the approaches discussed above. For example, the anti-antibody can be coupled to biotin which is detected by reacting with the streptavidin-horseradish peroxidase system discussed above.

One preferred embodiment utilizes biotin. The biotinylated antibody is in turn reacted with streptavidin-horseradish peroxidase complex. Orthophenylenediamine, 4-chloro-naphthol, or tetramethylbenzidine (TMB) can be used to effect chromogenic detection.

The preferred immunoassay format for practicing this invention is a forward sandwich assay in which the capture reagent has been immobilized, using conventional techniques, on the surface of the support.

Suitable supports used in assays include synthetic polymer supports, such as polypropylene, polystyrene, substituted polystyrene, e.g., aminated or carboxylated polystyrene; polyacrylamides; polyamides; polyvinylchloride, etc.; glass beads; agarose; nitrocellulose, etc.

As mentioned above, it is also possible to treat an individual suffering from a cancerous or precancerous condition, preferably the ones being diagnosed by the probes by supplying a therapeutic amount of the Wilms' Tumor suppressor gene product. This can be accomplished by a number of methods known in the art. Williams, D.A., et al, Nature 310:476-480 (1984); Cepko, C.L., et al, Cell 37(3):1053-1062 (1984). For example, one can use gene transfer techniques to prepare a vector containing a nucleotide sequence corresponding to the Wilms' tumor gene or functional fragment thereof and use such a vector to transform the malignant cells. The vector is preferably a retroviral vector such as described by Brown and Scott, in DNA Cloning, vol III, A Practical Approach, ch. 9, "Retroviral Vectors", CRL Press (1987).

Alternatively, one can use, for example, a Wilms' tumor transformed cell line to produce large amounts of the functional Wilms' tumor gene product which is then isolated and purified so that it is substantially free of pyrogens and endotoxins. The gene product is preferably purified so that is is at least about 90% pure, more preferably at least about 95% pure, still more preferably at least about 98% pure. The purified protein is then packaged by standard pharamaceutical procedures so that it can be delivered to the malignant cells, such as by injection, carrier-linked preparations, etc. A therapeutically effective amount of the purified protein is used. The therapeutically effective amount can readily be determined empirically based upon the present disclosure.

The invention will now be illustrated further by the following Exemplificaiton.

### Exemplification

### Materials and Methods

### Cell Culture

Somatic cell hybrids were isolated containing chromosome 11 or translocation chromosomes from patient cell lines DG-85-1436 and GM4613. DG85-1436 is a fibroblast cell line derived from a patient with familial aniridia involving a cytologically balanced translocation of chromosome 11 and 22 [t(11;22)(p13;q12.2)]. Moore, et al., Hum. Genet., 72:297-302 (1986). GM4613 is a fibroblast cell line (Human Genetic Mutant Cell Repository, Camden, NJ) exhibiting a cytologically balanced translocation involving chromosome 2 and 11 (t(2;11)(pll;p13)) derived from a neonate with Potter syundrome. Potter, In: Normal and Abnormal Development of the Kidney, Year Book Medical Publ., Chicago, IL., pp. 3-79, 83-123 and 259-281 (1972). Somatic cell hybrids were isolated as previously described. Glaser, et al., Nature, 321:282-887 (1986). The chromosome 11 haplotype of these hybrids was determined by RFLP analyses with DNA probes on both the short and long arms of chromosome 11. All initial DG hybrids retained the der (11), der (22), and the normal chromosome 11. Cell surface antigen studies revealed that a minor subpopulation of one hybrid, DG-7A-3, possessed only the der (22) chromosome. Two hybrids R19-2C and R19-3B, possessing only the der (22) chromosome were isolated by cell surface antigen selection from the DG-7A-3 population. This was accompanied by selecting for retention of the mer2 surface antigen in 11pl5 and selecting against the MICl surface antigen centromeric to the translocation in 11p13. In the case of the Potter patient, GM4613 hybrids which retained only the der (11) (BW G2-5), the der 2 (BW A2-5) or the normal 11 chromosome (BW H2-3) were identified by RFLP analysis.

Patient HV has familial aniridia associated with a cytologically balanced translocation involving chromosome 11 and 4 t(4;11)(q22;p13), as described by Simola, et al., Hum. Genet., 63:158-161 (1983). HV human-mouse hybrid R195 contains the der (11) chromosome and HV hybrid LHV-1A5 contains the der (4) chromosome. Hybrids from WAGR patients JH, MH and NW, have been described. Glaser, et al, Nature, 321:882-887 (1986). Mouse-human hybrid 15.14 hybrid from Wilms' tumor patient DR with an interstitial deletion of 11p13-p12 (Turleau, et al., Hum. Genet., 67:455-456 (1986)) has been characterized. Genomic DNA from this cell line was kindly provided by Dr. Claudine Junien (INSERM, Paris). Cell line WiT-13 was derived from xenograph cultures of a stage III Wilms' tumor with classical triphasic histology; the tumor arose sporadically in an otherwise healthy two year old female. Lewis, et al., Genomics, 3:25-31 (1988).

### Isolation of Cosmid Clones

High molecular weight DNA was prepared from the J1-11 hybrid, a Chinese hamster-human somatic cell hybrid possessing only the short arm of human chromosome 11.

Kao, et al., Proc. Natl. Acad. Sci. USA, 73:193-197 (1976). This DNA was used to construct cosmid libraries in the vectors pJB8 (Ish-Horowitz, et al., Nucl. Acid. Res., 9:2989-2998 (1981)) and pWe15 according to the method of Evans and Wahl. Evans, et al., Methods In Enzym., 152:604-610 (1987). DNA was partially digested with the restriction enzyme Mbo I and fragments of 35 to 45 kb were isolated using a 5-25% NaCl gradient. This DNA was ligated to vector DNA and packaged as phage (Gigapack Gold (Trade Mark), Stratagene, La Jolla, CA) which were used to infect E. coli strains 1046 or DH5. Colonies were plated at a low density (1,000 to 2,000 per 150mm plate) on LB-ampicillin plates. Maniatis, et al., Molecular Cloning: A Laboratory Manual**,** Cold Spring Harbor Laboratory, Cold Spring Harbor, NY (1982).

### Screening of Genomic Libraries

Standard screening methods were performed, as outlined by Maniatis, et al., (1982) ibid. Replica filters were screened for human positives by hybridization with radiolabeled total human DNA (Gusella, et al., Proc. Natl. Acad. Sci. USA, 77:2829-2833 (1988), a cloned human Alu repeat probe (Blur 11) (Jelinik, et al., Proc. Natl. Acad. Sci. USA, 77:1398-1402 (1980)) or Cot₁ human repeat enriched DNA (Shih, et al., Cell, 29:616-619 (1982)). Approximately 0.5-1% of colonies in the J1-11 library were identified as human positive. Cosmid DNA was isolated from small scale cultures of each of these human positive colonies according to Maniatis, et al., (1982) ibid. The EcoRI restriction pattern of cosmids was analyzed by standard agarose gel electrophoresis.

### Mapping of Cosmids

An abbreviated mapping panel of J1 cell hybrids possessing defined sgements of human chromosome 11p was used to rapidly identify human cosmids in 11p13. Human cosmids were mapped by preannealing radiolabelled DNA with total sheared human DNA to minimize signal from human repeats (Litt, et al., Proc. Natl. Acad. Sci. USA, 82:6206-6210 (1985)) and hyridizing with a nylon (Zetabind (Trade Mark), AMF-Cuno) filter of Eco RI digested DNA from J1 cell hybrid.

### Isolation of Single Copy Sequences

Single copy sequences were subcloned from cosmids as follows. Cosmids were digested to completion with Sau3A I and the resulting fragments subcloned into the Bam HI polylinker site of the plasmid pUC19. Clones with inserts were gridded on nitrocellulose filters and those with single copy sequences were identified by lack of hybridization to repeat enriched (Cot₁)DNA. Random single copy fragments were further tested by hybridizing radiolabelled inserts isolated from low melt agarose gel slices to nitrocellulose filters of human and λphage DNA. Probes J7-18p2 and J9-3p4 were among the single copy sequences identified from these cosmids.

### Origin of DNA Probes

Human cosmids J7-18, J8-3 and J10-15 were isolated from the J1-11/pWe15 cosmid library. Four additional cosmids (L156, L159, L155-1, L109) were isolated from a total human pWel5 cosmid library (Stratagene, La Jolla, CA) using a 1.8 kb Eco RI fragment of the WT33 cDNA as a probe. The localization of all cosmids to 11p13 was verified by somatic cell mapping. Genomic probes J7-18p2 and J8-3p4 were identified as 0.5 kb and 1.3 kb single copy Eco RI/HindIII fragments in pUC19 from cosmids J7-18 and J8-3, respectively. Phage K13 was isolated from a λdash (Stratagene) library constructed from a BamHI complete digest of Goss-Harris hybrid 3A. The CAT probe is a 0.6 kb PstI-AvaI fragment of the cDNA clone pC24. Bruns, et al., Am. J. Hum. Genet., 36:245 (1984). The FSHB probe is a 1.4 kb PstI insert of pFSH-1.4. Watkins, et al., DNA, 6:205-212 (1987).

### Southern Blots

Isolation and digestion of genomic DNA, transfer of DNA to nylon membranes, hybridization of radiolabelled probe, washing of filters, and autoradiography were performed as outlined by Glaser, et al., (1986) ibid. DNA was radiolabeled with ³²P-αdCTP (New England Nuclear) according to the random primer method. Feinberg, et al., Biochem. Biophys. Res. Comm., 111: 47-54 (1983).

### Screening of cDNA Libraries

Human cDNA libraries of embryonic kidney, adult kidney and pre-B cell origin were screened. Maniatis, et al., (1982) ibid. For screening each library, a total of 10⁶ phage were plated on NZCYM agarose plates and two replicas of each plate were made with nitrocellulose filters. Schleicher and Schull. The replica filters were treated with denaturing solution, neutralizing solution and 2X SCC (1X SCC= 0.15M NaCl, 0.015 M Na citrate) for 5 minutes each and then baked at 80°C in a vacuum oven for 2 hours according to Maniatis, et al., (1982) ibid. Replica filters were hybridized with the conserved single copy probe, J8-3p4, or with subfragments of WT cDNAs.

### Northern Blots

Total RNA was isolate by a LiCl/urea procedure. Auffray, et al., Eur. J. Biochem., 107:303-314 (1980). Cells wer harvested, pelleted, resuspended in 3M LiCl/6M urea and homogenized at 4°C. RNA was precipitated, washed in 3M LiCl/6M urea, precipitated and resuspended in TE/SDS. RNA was extracted in phenolchloroform (2-3x), ethanol precipitated, lyophilized, resuspended, quantitated and stored at -20°C. 10-20 µg of RNA was run on a 1% agarose 37% formaldehyde RNA gel and blotted on Gene Screen Plus (Trade Mark) (New England Biolabs) membrane. The filters were prehybridized and hybridized at 42°C for 24 hours in 50% formamide, 5x Denhardts solution, 0.5% SDS (sodium dodecyl sulfate), 10% dextran sulfate, 0.1% pyrophosphate and 100 µg/ml salmon sperm DNA. Blots were hybridized with a conserved genomic probe J8-3p4, cDNA 2-1 (1.5 kb Pst I/Eco RI fragment), a 0.5 kb Sau3a I subclone of cDNA 2-1 or a 1.8 kb Eco RI fragment of the cDNA WT33. After an 18-36 hour hybridization, blots were washed twice in 2xSSC. 0.1% SDS for 30 minutes at room temperature and one to two times in 1xSSC, 0.1% SDS for 30 minutes at 55-60°C.

### DNA Sequencing

DNA sequencing was done by chain termination, Sanger, et al., Proc. Nat. Acad. Sci. USA, 74:5463-5467 (1977) using double stranded DNA templates. Restriction fragments of the WT33 cDNA were subcloned into pUC19 or Bluescript (Trade Mark) (New England Biolabs). Direct sequencing primers for Bluescript were obtained from New England Biolabs. Additional oligonucleotide primers (synthesized by Research Genetics, Huntsville, AL), corresponding to the cDNA were also used for sequencing the cDNAs. The WT33 cDNA was sequenced on both strands. In addition, sequence was confirmed on regions of other cDNAs (WT2, WT4 and WT22). Sequencing reactions were electrophoresed on 6% and 8% polyacrylamide gels, dried and autoradiographed. Using the Fast-P algorithm. Lipman, et al., Science, 227:1435-1441 (1985), the predicted amino acid sequence of the cDNA WT33 was compared with protein sequences stored in the National Biomedical Research Foundation Protein Identification Resource (NBRF/PIR data base).

### Results

### Isolation And Mapping Of Genomic Probes

A cosmid library was constructed from a hybrid clell line, J1-11, in which the short arm of chromosome 11 had been segregated from the remainder of the human genome in a Chinese hamster background. Kao et al., (1976) ibid. A total of 119 cosmid clones containing human DNA sequences were isolated, all of which mapped to the short arm of chromosome 11. To identify clones within the WAGR region, a mapping panel of somatic cell hybrids containing different fragments of human chromosome 11p was used. Glaser, et al., (1989) ibid. Three cosmids, J7-18, J8-3 and J10-15, appeared to map the closest to the region containing the Wilms' tumor gene. The restriction maps of cosmids J8-3 and J10-15 showed substantial overlap. Hence, single copy sequences (J7-18p2 and J8-3p4) were subcloned and identified from cosmids J7-18 and J8-3, respectively.

The fine localizatin of these single copy DNA sequences within 11p13 was determined by hybridization to a series of somatic cell hybrids derived from patients with translocations and deletions which define specific intervals within the WAGR region. Subclones J7-18p2 and J8-3p4 were hybridized to DNA from hybrid cells derived from an aniridia patient DG. This patient has a cytogenetically balanced 11;22 translocation which bisects band 11p13. The translocation was inherited with aniridia for several generations in this family, Moore, et al., (1986) ibid, and is associated with a small molecular deletion at the breadpoint. Davis, et al., (1988b) ibid.; Gessler, et al., (1989b) ibid. Human DNA sequences homologous to probes J7-18p2 and J8-3p4 were shown to be absent in cell lines R19-2C and R19-3B, which contain only the derivative (der) (22) chromosome. These results, and a normal gene dosage in fibroblast DNA from this patient, place J7-18p2 and J8-3p4 on the centromeric side of the DG translocation breakpoint on chromosome 11. Analogous results were obtained with hybrid cells derived from a second unrelated aniridia patient (HV) also carrying an 11p13 translocation (Table). Accordingly, both cosmids map centromeric to AN2, towards the Wilms' tumor locus.

**Table**

| Mapping of 11p13 Probes Using Patient Hybrids | | | | | | |
|---|---|---|---|---|---|---|
| Phenotype | Patient | | Chromosome 11 | Probe | | cDNA |
| | | Hybrid | Content | J7-18p12 | J8-3p4 | |
| Aniridia | DG | R19-2C | der(22) | - | - | - |
| | HV | R195 | der(11) | ND | + | + |
| | HV | LHV-1A5 | der(4) | - | - | - |
| | | | | | | |
| Urogenital | BW | H2-3 | N1(11) | + | + | + |
| Defects | BW | G2-5 | der(11) | - | - | - |
| | BW | A2-5 | der(2) | + | + | + |
| | | | | | | |
| WAGR | JH | C/h | del(11p14.1-p11.2) | | - | - |
| | NW | F3 | del(llpl3) | - | - | - |
| | MJ | A9 | del(11p13) | - | - | - |
| | | | | | | |
| WT (constitutional) | DR | 15.14 | del(11)p13-p12) | + | - | - |
| | | | | | | |
| WT (sporadic) | WiT-13 | D2 & R87 | del(11)pΔS) | + | - | - |
| | WiT-13 | R91 | del(11)pΔL) | - | - | - |

Hybridization of J7-18p2 to DNA from somatic cell hybrids derived from a second patient (BW), an individual with multiple urogenital defects (Potter's syndrome) and a (t(2;11)(pll;p13) translocation (GM4613; Human Genetic Muatant Cell Repository, Camden, NJ) was hybridized to J7-18p2 and J8-3p4. The breakpoint of this translocation identifies the site of a potential genetic determinant of genitourinary abnormalities. Porteus, et al., (1987) ibid. Both probes hybridize to cell line A2-5, containing the det (2) chromosome and fail to hybridize to cell line G2-5, containing the der (11) chromosome. Therefore, both are located between the aniridia and Potter translocation breakpoints. Since this interval contains the Wilms' tumor gene, these findings suggest that J7-18p2 and J8-3p4 are close to or within the Wilms' tumor locus.

Analysis of chromosome 11 deletions from WAGR and Wilms' tumor patients (Table) permits more precise localization of these probes in relation to the Wilms' tumor gene. Both J7-18p2 and J8-3p4 are hemizygously deleted in three constitution WAGR deletions (patients JH, MJ and NW) tested, consistent with the positioning of these DNA sequences in close proximity to the Wilms' tumor locus.

The position of J7-18p2 and J8-3p4 relative to the Wilms' tumor locus was further investigated by hybridization to DNA from cell lines derived from two Wilms' tumor patients. Patient DR is an individual with a constitutional deletion of 11p12-p13, Couillin, et al., (1988) ibid., terminating between the Wilms' tumor and AN2 loci (Table). J7-18p2 is present in the deleted chromosome 11 of patient DR, whereas J8-3p4 is absent (Table). Since catalase is deleted in DR, J7-18p2 must be telomeric to J8-3p4. The distance between these two probes is less than 340 kb. The DR data indicates that the distal boundary of the region which must contain the Wilms' tumor patient WiT-13 has previously been shown, Lewis, et al., (1988) ibid., to carry overlapping 11p13 deletions in tumor tissue, as demonstrated by the homozygous deletion of the anonymous DNA segment D11S87. Since J7-18p2 is present in this chromosome, but J8-3p4 is absent (Table), the Δ_{S} (small) deletion chromosome 11 of WiT-13 must have a breakpoint between the two probes. J8-3p4 is homozygously deleted in WiT-13 since it also was found to be absent in the Δ_{L} (large) deletion chromosome. The proximal limit to the position of the Wilms' tumor locus is the endpoint of the Δ_{L} deletion on WiT-13. The finding that J8-3p4 is homozygously deleted in WiT-13, thus, maps it to the 11p13 region containing the Wilms' tumor locus, an interval which is 345 kb or less based on analysis by pulsed field gel electrophoresis. A map summarizing these findings is shown in Figure 2.

### Isolation Of cDNA Clones

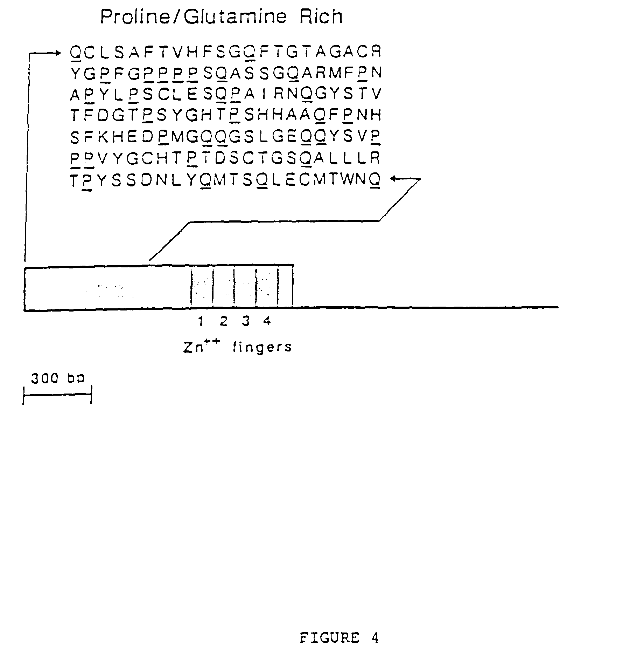

The map position of J8-3p4 indicated that this probe was close to or within the Wilms' tumor locus. Two observations suggested that J8-3p4 contained a portion of a transcription unit. First, strong cross-species hybridization to hamster and mouse DNA genomic sequences was observed in somatic cell hybrids with J8-3p4 (Figures 1A and 1B). Cross species conservation is often associated with expressed DNA sequences. Second, J8-3p4 showed hybridization to RNA isolated from baboon kidney and spleen. J8-3p4 was used as a probe to screen a cDNA library derived from human embryonic kidney (HEK) cells. On the basis of Northern blotting results, a human adult kidney and a human pre B cell library were also screened. Four cDNA clones, two from HEK (WT4, WT2) one from human adult kidney (WT22) and one from a pre B cell line (WT33), were studied in detail. Using another independently isolated conserved genomic DNA clone, λK13, a fifth homologous cDNA clone (WT13) was also isolated from the HEK library. The longest cDNA clone isolated, WT33, is 2313 base pairs in length (Figure 3 and 4). The WT33 cDNA extends the furthest in both the 5' and 3' directions. The other four cDNAs share a common internal region of DNA sequence approximately 1000 to 1200 base pairs in length.

### Sequence Analysis Of The WT33 cDNA

The nucleotide sequence of the WT33 cDNA was determined and the predicted amino acid sequence was derived. The sequence of WT33 reveals a continuous open reading frame of 345 amino acids which extends from nucleotide 1 to 1035. A schematic representation of the WT33 cDNA is illustrated in Figure 3. This open reading frame represents most of the WT33 coding segment, but it does not include the initiator methionine codon. Primer extension experiments suggest that an additional 200 bp are present at the 5' end of the mRNA corresponding to WT33. The transcription pattern of the locus corresponding to WT33. The transcription pattern of the locus corresponding to these cDNAs exhibits some complexity. Experiments utilizing RNA PCR (polymerase chain reaction) indicate variation in mRNA sequence in the 5' segment of the coding region of the mRNA, suggesting alternative splicing patterns among various tissue types.

Of particular interest, nucleotide 670 to 1002 encode four contiguous "zinc finger" domains. The zinc finger motif was first described in Xenopus TF-IIIA which binds to DNA in the internal control region of the 5S genes. Miller, et al, EMBO. J., 4:1609-1614 (1985); Brown, et al., FEBS Lett., 186:271-274 (1985). Subsequently, other nucleic acid recognizing proteins have been reported to contain the zinc finger domain. Klug, et al., TIBS, 12:464 (1987); Evans, et al., Cell, 52:1-3 (1988). The zinc finger sequence motif consists of a repeating unit of 29-30 amino acids (Y/F-X-C-X₂₋₄-C-X₃-F-X₅-L-X₂-H-X₃₋₄-H-X₆₋₇; where X is any amino acid) which folds into a domain chelating a zinc atom between a pair of cysteines and histidines. Diakun, et al., Nature, 324:698-699 (1986); Green, et al., Proc. Natl. Acad. Sci. USA, 86:4047-4051 (1989). All four zinc fingers encoded by WT33 (Figures 4 and 5) fit the consensus sequence for zinc fingers. The H/C link between zinc fingers, typified by the amino acid sequence TGE-R/K-P-F/Y-X. Suh, et al., Cell, 47:1025-1032 (1986), is also conserved in the deduced amino acid sequence of WT33.

### Bacterial Expression of Wilms' Tumor Gene Product

A glutathione S-transferase-Wilms' Tumor fusion protein was generated by insertion of a fragment of the Wilms'cDNA into the bacterial expression vector pGEX-3X (Pharmacia). The plasmid WT33 was digested with Bam H1 and rendered blunt with Klenow followed by restriction with Rsr II. The resulting fragment was ligated to a DNA linker (McGill University Dept. of Biochemistry) containing a stop codon and subcloned into the blunted Bam H1 site of pGEX-3X. The resulting plasmid pGEX/h W.T. was introduced into E. coli strain NB42. This plasmid is capable of expressing a fusion protein that contains amino acids 97-295 (numbers refer to Figure 3) of the Wilms' tumor protein.

Growth induction and isolation of the fusion protein was performed according to the manufacturers instructions. Expression was analyzed by SDS-PAGE.

### Results

### Bacterial Expression

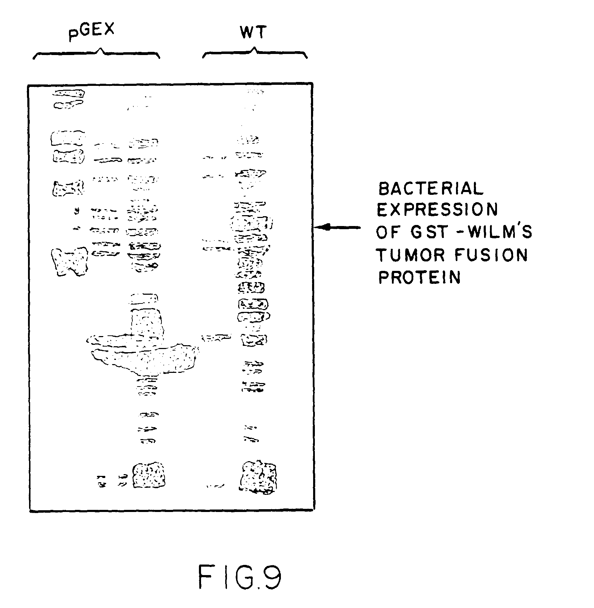

A plasmid encoding a glutathione S-transferase (GST) Wilms' Tumor fusion protein (GST-WT) containing amino acids 95-295 of the Wilms' Tumor protein was generated. Figure 9 shows that this plasmid is capable of expressing a ca. 50 kDa protein. Lanes 1 and 3 labeled pGEX show the expression of the GST portion of the fusion protein that is expressed by the vector. This protein is soluble and binds efficiently to Glutathione sepharose (Pharmacia #17-0756-01). [Lanes 1 and 4 are supernatant, Lanes 2 and 5 are pellet, Lanes 3 and 6 are Glutathione-Affinity]. The arrow shows the expresson of the ca. 50 kDa fusion protein. In contrast to the GST portion alone, the GST-WT protein is insoluble and can not be purified by glutathione sepharose affinity chromatography. See Lanes 3 and 6.

## Claims

1. Isolated DNA comprising the 11p13 Wilms' tumor gene, or a portion thereof of at least 20 nucleotides, the gene consisting of the nucleotide sequence shown in Figure 3, or a substantially homologous DNA which codes for a protein that, in its active form, has the same biological activity as that encoded by the nucleic acid sequence of Figure 3.

2. An isolated polypeptide product of the 11p13 Wilms' tumor gene encoded by the DNA as defined in claim 1, or an antigenic portion thereof.

3. An antibody (e.g. a monoclonal antibody) specific for the polypeptide product as defined in claim 2.

4. An antibody (e.g. a monoclonal antibody) specific for a polypeptide which has all or an antigenic portion of the amino acid sequence of Figure 3.

5. A method of detecting Wilms' tumor DNA in a cell, comprising the steps of:
(a) rendering DNA present in the cell available for hybridization with complementary DNA;
(b) contacting DNA produced in step (a) with a DNA probe (e.g. a detectably labelled DNA probe) which is a nucleotide sequence complementary to all or a portion of Wilms' tumor gene as defined in claim 1, or a substantially homologous nucleotide sequence, under conditions appropriate for hybridization of complementary nucleotide sequences to occur; and
(c) detecting hybridization of DNA present in the cell with the DNA probe wherein hybridization of DNA present in the cell with DNA probe is indicative of presence of Wilms' tumor DNA.

6. The method of claim 5 wherein the nucleotide sequence of the DNA probe is all or a portion of the nucleotide sequence of Figure 3, and for example;
(a) the nucleotide sequence of the DNA probe is the portion encoding a polypeptide containing a zinc finger; or
(b) wherein the nucleotide sequence of the DNA probe corresponds to the nucleotide sequence which encodes a peptide containing one of the WT33 zinc fingers shown in Figure 3; or
(c) wherein the nucleotide sequence of the DNA probe is a portion of the nucleotide sequence encoding the proline/glutamine rich region, and for example the proline/glutamine rich region is that shown in Figure 4.

7. A method of detecting alterations of the Wilms' tumor gene in a human which comprises testing a cell or biological fluid from the human for the presence of unaltered Wilms' tumor gene or gene product by:
(a) contacting the cell or fluid with at least one probe, which is capable of binding either with Wilms' tumor gene as defined in claim 1 or a substantially homologous nucleotide sequence, or with the Wilms' tumor gene polypeptide product as defined in claim 2;
(b) determining whether binding has occurred.

8. An immunoassay for detecting or quantifying the presence of Wilms' tumor gene as defined in claim 1, in a cell or biological fluid obtained from a human, which comprises:
(a) reacting the cell or fluid with at least one first monoclonal antibody which is capable of binding to the Wilms' tumor gene polypeptide product as defined in claim 2;
(b) reacting the product of step (a) with at least one detectably labelled second monoclonal antibody, which is capable of binding to Wilms' tumor gene product at an epitope different from the epitope bound by the first antibody; and
(c) detecting or quantifying the product of step (b).

9. The assay according to claim 8 wherein
(a) immunoreactive antibody fragments are used; or
(b) the detectable label is selected from the group consisting of radioisotopes, enzymes, fluorogenic, chemiluminescent and electrochemical materials; or
(c) the second antibody is conjugated to biotin.

10. A method of assay for the presence of cancer or precancerous condition in a human cell, which comprises:
(a) taking a predetermined cell or fluid sample from a human;
(b) adding a probe for the human Wilms' tumor gene or polypeptide product as defined in claim 1 or claim 2 respectively to the predetermined cell or fluid sample;
(c) determining from the interaction of the probe with its target(s) whether there has been an alteration or deletion of a functional portion of the Wilms' tumor gene in the predetermined cell or fluid sample wherein an alteration or deletion of the Wilms' tumor gene or its polypeptide product is indicative of the cancer or precancerous condition.

11. The method of claim 10 wherein
(a) the probe is a nucleotide probe; or
(b) the probe is used to determine whether there has been a deletion; or
(c) the probe is a probe for a deletion in a chromosome containing the Wilms' tumor gene; or
(d) the probe is an antibody probe.

12. The method of claim 11 wherein the probe corresponds to at least 20 nucleotides from the Wilms' tumor gene shown in Figure 3, and for example wherein the method of determining whether there is a deletion involves the use of polymerase chain reaction.

13. The polypeptide product of the Wilms' tumor gene as defined in claim 2 for use in therapy e.g. treating a cancer or precancerous condition caused by an alteration or deletion of the Wilms' tumor gene.

14. A retroviral vector containing a functional Wilms' tumor gene as defined in claim 1 for use in therapy e.g. in adding the Wilms' tumor gene product of claim 13 to diseased cells by gene transfer technology.

15. A vector containing a nucleotide segment containing:
(a) a sufficient number of nucleotides corresponding to a functional human Wilms' tumor gene as defined in claim 1 or functional fragment thereof to express a protein having Wilms' tumor suppressor activity wherein the vector does not contain an entire human chromosome, and
(b) a promoter upstream of the nucleotide segment.

16. The vector of claim 15 wherein the promoter is a viral promoter and the vector also contains an enhancer and polyadenylation sequences.

17. A method of quantitating Wilms' tumor DNA in a cell, comprising the steps of:
(a) rendering DNA present in the cell available for hybridization with complementary DNA;
(b) contacting DNA produced in step (a) with a detectably labelled DNA probe which is a nucleotide sequence complementary to all or a portion of Wilms' tumor DNA, the nucleotide sequence of said DNA or portion thereof being all or a portion of the nucleotide sequence shown in Figure 3, or a substantially homologous nucleotide sequence, under conditions appropriate for hybridization of complementary DNA sequences to occur and formation of Wilms' tumor DNA/detectable labelled DNA probe complexes; and
(c) determining the quantity of the Wilms' tumor DNA/detectably labelled DNA probe complexes.

18. A DNA probe for use in the method of claim 17, (a) which is complementary to the nucleotide sequence of Figure 3, or a portion thereof; or (b) consisting essentially of the nucleotide sequence of Figure 3, or a portion thereof.

19. A method of detecting Wilms' tumor gene-encoded polypeptide in a sample obtained from an individual, comprising the steps of:
(a) combining the sample with at least one antibody specific for a polypeptide which has all or a portion of the amino acid sequence of Figure 3, under conditions appropriate for binding or Wilms' tumor gene-encoded polypeptide and the antibody and formation of Wilms' tumor gene-encoded polypeptide/antibody complexes; and
(b) detecting Wilms' tumor gene-encoded polypeptide/antibody complexes.

20. The method of claim 19 further comprising quantitating the Wilms' tumor gene-encoded polypeptide/antibody complexes by determining the quantity of detectably labelled antibody present in said complex.

21. A kit for detecting Wilms' tumor gene as defined in claim 1, comprising:
(a) a DNA probe which is a nucleotide sequence complementary to all or a portion of the Wilms' tumor gene; and
(b) a container.

22. A kit for detecting Wilms' tumor gene-encoded polypeptide product in a cell, comprising:
(a) an isolated antibody specific for a polypeptide product as defined in claim 2; and
(b) a container.

## Patentansprüche

1. Isolierte DNS, die das 11p13 Wilms-Tumor-Gen oder einen Teil davon mit mindestens 20 Nucleotiden umfaßt, wobei das Gen aus der in Figur 3 gezeigten Nucleotid-Sequenz oder einer im wesentlichen homologen DNS besteht, die für ein Protein codiert, das, in seiner aktiven Form, die selbe biologische Aktivität wie das von der Nucleinsäure-Sequenz von Figur 3 codierte Protein besitzt.

2. Ein isoliertes Polypeptid-Produkt des 11p13 Wilms-Tumor-Gens, das von der in Anspruch 1 definierten DNS codiert ist, oder ein antigener Teil davon.

3. Ein Antikörper (z.B. monoklonaler Antikörper), der für das in Anspruch 2 definierte Polypeptid-Produkt spezifisch ist.

4. Ein Antikörper (z.B. monoklonaler Antikörper), der für ein Polypeptid spezifisch ist, das die gesamte oder einen antigenen Teil der Aminosäure-Sequenz von Figur 3 besitzt.

5. Ein Verfahren zur Detektion von Wilms-Tumor-DNS in einer Zelle, das die Schritte umfaßt:
(a) Verfügbar-Machen von in der Zelle vorhandener DNS für eine Hybridisierung mit komplementärer DNS;
(b) In-Kontakt-Bringen von in Schritt (a) hergestellter DNS mit einer DNS-Sonde (z.B. einer detektierbar markierten DNS-Sonde), die eine zum gesamten oder zu einem Teil des in Anspruch 1 definierten Wilms-Tumor-Gens oder zu einer im wesentlichen homologen Sequenz komplementäre Nucleotid-Sequenz ist, unter Bedingungen, die für eine zu ereignende Hybridisierung komplementärer Nucleotid-Sequenzen geeignet sind; und
(c) Detektion der Hybridisierung von in der Zelle vorhandener DNS mit der DNS-Sonde, worin eine Hybridisierung von in der Zelle vorhandener DNS mit der DNS-Sonde ein Hinweis auf Vorhandensein von Wilms-Tumor-DNS ist.

6. Das Verfahren nach Anspruch 5, worin die Nucleotid-Sequenz der DNS-Sonde die gesamte oder ein Teil der Nucleotid-Sequenz von Figur 3 ist, und zum Beispiel:
(a) die Nucleotid-Sequenz der DNS-Sonde der Teil ist, der ein Polypeptid codiert, das einen Zinkfinger enthält; oder
(b) worin die Nucleotid-Sequenz der DNS-Sonde der Nucleotid-Sequenz entspricht, die ein Peptid codiert, das einen der in Figur 3 gezeigten WT33-Zinkfinger enthält; oder
(c) worin die Nucleotid-Sequenz der DNS-Sonde ein Teil der Nucleotid-Sequenz ist, die die Prolin/Glutamin reiche Region codiert und zum Beispiel die Prolin/Glutamin reiche Region die ist, die in Figur 4 gezeigt ist.

7. Ein Verfahren zur Detektion von Veränderungen des Wilms-Tumor-Gens in einem Menschen, das ein Testen einer Zelle oder biologischer Flüssigkeit vom Menschen auf das Vorhandensein eines unveränderten Wilms-Tumor-Gens oder Gen-Produkts umfaßt mittels:
(a) In-Kontakt-Bringen der Zelle oder Flüssigkeit mit mindestens einer Sonde, die in der Lage ist, entweder mit dem in Anspruch 1 definierten Wilms-Tumor-Gen oder einer im wesentlichen homologen Nucleotid-Sequenz oder mit dem in Anspruch 2 definierten Wilms-Tumor-Gen Polypeptid-Produkt zu binden;
(b) Bestimmen, ob ein Binden erfolgt ist.

8. Ein Immuntest zur Detektion oder quantitativen Bestimmung auf das Vorhandensein eines in Anspruch 1 definierten Wilms-Tumor-Gens in einer Zelle oder biologischen Flüssigkeit, die von einem Menschen erhalten ist, wobei der Immuntest umfaßt:
(a) Reaktion der Zelle oder Flüssigkeit mit mindestens einem ersten monoklonalen Antikörper, der in der Lage ist, an das in Anspruch 2 definierte Wilms-Tumor-Gen Polypeptid-Produkt zu binden;
(b) Reaktion des Produkts von Schritt (a) mit mindestens einem detektierbar markierten zweiten monoklonalen Antikörper, der in der Lage ist, an ein Wilms-Tumor-Gen Produkt an einem anderen Epitop als das vom ersten Antikörper gebundene Epitop zu binden; und
(c) Detektion oder quantitative Bestimmung des Produkts von Schritt (b).

9. Der Test gemäß Anspruch 8, worin
(a) immunreaktive Antikörper-Fragmente verwendet sind; oder
(b) der detektierbare Marker aus der Gruppe ausgewählt ist, die aus Radioisotopen, Enzymen, fluororganischen, chemilumineszenten und elektrochemischen Stoffen besteht; oder
(c) der zweite Antikörper an Biotin konjugiert ist.

10. Ein Verfahren zum Testen auf das Vorhandensein eines Karzinoms oder präkanzerösen Zustands in einer menschlichen Zelle, das umfaßt:
(a) Entnahme einer zuvor bestimmten Zelle oder flüssigen Probe von einem Menschen;
(b) Zugabe einer Sonde für das in Anspruch 1 definierte Wilms-Tumor-Gen oder das in Anspruch 2 definierte Polypeptid-Produkt zu der zuvor bestimmten Zelle oder flüssigen Probe;
(c) Bestimmung aus der Wechselwirkung der Sonde mit ihrem/ihren Ziel(en), ob es eine Veränderung oder Deletion eines funktionalen Teils des Wilms-Tumor-Gens in der zuvor bestimmten Zelle oder flüssigen Probe gegeben hat, worin eine Veränderung oder Deletion des Wilms-Tumor-Gens oder dessen Polypeptid-Produkts ein Hinweis auf das Karzinom oder den präkanzerösen Zustand ist.

11. Das Verfahren nach Anspruch 10, worin
(a) die Sonde eine Nucleotid-Sonde ist; oder
(b) die Sonde verwendet ist, um zu bestimmen, ob es eine Deletion gegeben hat; oder
(c) die Sonde eine Sonde für eine Deletion in einem das Wilms-Tumor-Gen enthaltenden Chromosom ist; oder
(d) die Sonde eine Antikörper-Sonde ist.

12. Das Verfahren nach Anspruch 11, worin die Sonde mindestens 20 Nucleotiden des in Figur 3 gezeigten Wilms-Tumor-Gens entspricht, und zum Beispiel worin das Verfahren zur Bestimmung, ob es eine Deletion gibt, die Verwendung der Polymerase-Kettenreaktion mit einschließt.

13. Das in Anspruch 2 definierte Polypeptid-Produkt des Wilms-Tumor-Gens zur Verwendung in einer Therapie, z.B. Behandlung eines Karzinoms oder präkanzerösen Zustands, das/der von einer Veränderung oder Deletion des Wilms-Tumor-Gens verursacht ist.

14. Ein retroviraler Vektor, der ein funktionales in Anspruch 1 definiertes Wilms-Tumor-Gen enthält, zur Verwendung in einer Therapie, z.B. Zugabe des Wilms-Tumor-Gen Produkts nach Anspruch 13 zu erkrankten Zellen mittels Gen-Transfer-Technik,

15. Ein Vektor, der einen Nucleotid-Abschnitt enthält, der enthält:
(a) eine hinreichende Anzahl an Nucleotiden, die einem funktionalen, in Anspruch 1 definierten Wilms-Tumor-Gen oder funktionalen Fragment davon entsprechen, um ein Protein mit Wilms-Tumor Suppressor-Aktivität zu exprimieren, worin der Vektor kein vollständiges menschliches Chromosom enthält, und
(b) einen Promotor stromaufwärts des Nucleotid- Abschnitts.

16. Der Vektor nach Anspruch 15, worin der Promotor ein viraler Promotor ist und der Vektor ebenfalls einen Enhancer und Polyadenylierungssequenzen enthält.

17. Ein Verfahren zur quantitativen Bestimmung von Wilms-Tumor-DNS in einer Zelle, das die Schritte umfaßt:
(a) Verfügbar-Machen von in der Zelle vorhandener DNS für eine Hybridisierung mit komplementärer DNS;
(b) In-Kontakt-Bringen von in Schritt (a) hergestellter DNS mit einer detektierbar markierten DNS-Sonde, die eine zu der gesamten oder einem Teil der Wilms-Tumor-DNS komplementäre Nucleotid-Sequenz ist, wobei die Nucleotid-Sequenz der besagten DNS oder eines Teil davon die gesamte oder ein Teil der in Figur 3 gezeigten Nucleotid-Sequenz oder eine im wesentlichen homologe Nucleotid-Sequenz ist, unter Bedingungen, die für eine zu ereignende Hybridisierung komplementärer DNS-Sequenzen geeignet sind, und Bildung von Wilms-Tumor-DNS/detektierbar markierte DNS-Sonde Komplexen; und
(c) Bestimmung der Menge der Wilms-Tumor-DNS/detektierbar markierte DNS-Sonde Komplexe.

18. Eine DNS-Sonde zur Verwendung in dem Verfahren nach Anspruch 17, (a) die zu der Nucleotid-Sequenz von Figur 3 oder einem Teil davon komplementär ist; oder (b) im wesentlichen aus der Nucleotid-Sequenz von Figur 3 oder einem Teil davon besteht.

19. Ein Verfahren zum Detektieren eines Wilms-Tumor-Gen codierten Polypeptids in einer Probe, die von einem Individuum erhalten ist, wobei das Verfahren die Schritte umfaßt:
(a) Vereinigen der Probe mit mindestens einem Antikörper, der für ein Polypeptid spezifisch ist, das die gesamte oder einen Teil der Aminosäure-Sequenz von Figur 3 besitzt, unter Bedingungen, die für ein Binden von Wilms-Tumor-Gen codiertem Polypeptid und dem Antikörper geeignet sind und Bildung von Wilms-Tumor-Gen codiertes Polypeptid/Antikörper Komplexen; und
(b) Detektion von Wilms-Tumor-Gen codierten Polypeptid/Antikörper Komplexen.

20. Das Verfahren nach Anspruch 19, das des weiteren eine quantitative Bestimmung der Wilms-Tumor-Gen codierten Polypeptid/Antikörper Komplexe mittels quantitativer Bestimmung von detektierbar markiertem Antikörper, der in besagtem Komplex vorhanden ist, umfaßt.

21. Ein Set zur Detektion von in Anspruch 1 definiertem Wilms-Tumor-Gen, wobei das Set umfaßt:
(a) eine DNS-Sonde, die eine zum gesamten oder einem Teil des Wilms-Tumor-Gens komplementäre Nucleotid-Sequenz ist; und
(b) einen Behälter.

22. Ein Set zur Detektion von Wilms-Tumor-Gen codiertem Polypeptid-Produkt in einer Zelle, wobei das Set umfaßt:
(a) einen isolierten Antikörper, der für ein in Anspruch 2 definiertes Polypeptid-Produkt spezifisch ist; und
(b) einen Behälter.

## Revendications

1. ADN isolé comprenant le gène 11p13 de la tumeur de Wilms, ou une portion de celui-ci d'au moins 20 nucléotides, le gène consistant en la séquence nucléotidique présentée à la figure 3, ou un ADN essentiellement homologue qui code pour une protéine qui, dans sa forme active, a la même activité biologique que celle codée par la séquence d'acide nucléique de la figure 3.

2. Produit polypeptidique isolé du gène 11p13 de la tumeur de Wilms, codé par l'ADN tel défini dans la revendication 1, ou une portion antigénique de celui-ci.

3. Anticorps (tel qu'un anticorps monoclonal), spécifique du produit polypeptidique tel que défini dans la revendication 2.

4. Anticorps (tel qu'un anticorps monoclonal), spécifique pour un polypeptide qui a toute la séquence d'acides aminés de la figure 3 ou une portion antigénique de celle-ci.

5. Méthode de détection de l'ADN de la tumeur de Wilms dans une cellule, comprenant les étapes consistant à :
(a) rendre l'ADN présent dans la cellule disponible pour l'hybridation avec l'ADN complémentaire ;
(b) mettre en contact l'ADN produit à l'étape (a) avec une sonde ADN (telle qu'une sonde ADN marquée de manière détectable) qui est une séquence nucléotidique complémentaire du gène entier ou d'une portion du gène de la tumeur de Wilms tel que défini dans la revendication 1, ou une séquence nucléotidique essentiellement homologue, dans des conditions appropriées pour l'hybridation des séquences nucléotidiques complémentaires ; et
(c) détecter l'hybridation d'ADN présent dans la cellule avec la sonde ADN, l'hybridation de l'ADN présent dans la cellule avec la sonde ADN étant indicatrice de la présence de l'ADN de la tumeur de Wilms.

6. Méthode selon la revendication 5 dans laquelle la séquence nucléotidique de la sonde ADN est toute la séquence nucléotidique de la figure 3 ou une portion de celle-ci, et par exemple ;
(a) la séquence nucléotidique de la sonde ADN est la portion d'ADN codant pour un polypeptide contenant un doigt de zinc ; ou
(b) la séquence nucléotidique de la sonde ADN correspond à la séquence nucléotidique qui code pour un peptide contenant les doigts de zinc WT33 présentés à la figure 3 ; ou
(c) la séquence nucléotidique de la sonde ADN est une portion de la séquence nucléotidique codant pour la région riche en proline/glutamine et par exemple la région riche en proline/glutamine est celle présentée à la figure 4.

7. Méthode de détection des altérations du gène de la tumeur de Wilms chez un humain, qui comprend le test d'une cellule ou d'un liquide biologique d'un humain pour la présence du gène ou du produit du gène de la tumeur de Wilms non altéré par les étapes consistant à :
a) mettre en contact de la cellule ou du liquide avec au moins une sonde, qui est capable de se lier soit avec le gène de la tumeur de Wilms tel que défini dans la revendication 1 ou une séquence nucléotidique essentiellement homologue, soit avec le produit polypeptidique du gène de la tumeur de Wilms tel que défini dans la revendication 2 ; et
(b) déterminer si la liaison a eu lieu.

8. Immunoessai pour la détection ou la quantification de la présence du gène de la tumeur de Wilms tel que défini dans la revendication 1, dans une cellule ou un liquide biologique obtenue à partir d'un humain, qui comprend les étapes consistant à :
(a) faire réagir la cellule ou le liquide avec au moins un premier anticorps monoclonal qui est capable de se lier au produit polypeptididique du gène de la tumeur de Wilms tel que défini dans la revendication 2 ;
(b) faire réagir le produit de l'étape (a) avec au moins un second anticorps monoclonal marqué de manière détectable, qui est capable de se lier au produit du gène de la tumeur de Wilms à un épitope différent de l'épitope lié au premier anticorps ; et
(c) détecter ou quantifier le produit de l'étape (b).

9. Essai selon la revendication 8 dans lequel :
(a) des fragments d'anticorps immunoréactifs sont utilisés ; ou
(b) le marquage détectable est choisi parmi des radioisotopes, des enzymes, des matériaux fluorogènes, fluoluminescents et électrochimiques ; ou
(c) le second anticorps est conjugué à de la biotine.

10. Méthode d'essai pour la présence d'un cancer ou d'un état précancéreux dans une cellule humaine, qui comprend les étapes consistant à :
(a) prélever une cellule ou un échantillon de liquide prédéterminé chez un humain ;
(b) ajouter une sonde pour le gène humain de la tumeur de Wilms, ou pour le produit polypeptidique tels que définis dans la revendication 1 ou la revendication 2 respectivement à la cellule ou à l'échantillon de liquide prédéterminé ;
(c) déterminer à partir de l'interaction de la sonde avec ses(sa) cible(s) s'il y a eu une altération ou une délétion de la portion fonctionnelle du gène de la tumeur de Wilms dans la cellule ou l'échantillon de liquide prédéterminé, une altération ou une délétion du gène de la tumeur de Wilms ou de son produit polypeptidique étant indicatif d'un cancer ou d'un état précancéreux.

11. Méthode selon la revendication 10 dans laquelle :
(a) la sonde est une sonde nucléotidique ; ou
(b) la sonde est utilisée pour déterminer s'il y a lieu une délétion ; ou
c) la sonde est une sonde destinée à mettre en évidence une délétion dans un chromosome contenant le gène de la tumeur de Wilms ; ou
d) la sonde est une sonde anticorps.

12. Méthode selon la revendication 11 dans laquelle la sonde correspond à au moins 20 nucléotides du gène de la tumeur de Wilms représenté à la figure 3, et par exemple dans laquelle la méthode de détermination d'une délétion implique l'utilisation de réaction en chaîne par la polymérase.

13. Produit polypeptidique du gène de la tumeur de Wilms tel que défini dans la revendication 2 pour une utilisation en thérapie, par exemple dans le traitement du cancer ou d'un état précancéreux causé par une altération ou une délétion du gène de la tumeur de Wilms.

14. Vecteur rétroviral contenant un gène fonctionnel de la tumeur de Wilms tel que défini dans la revendication 1, pour une utilisation en thérapie par exemple en ajoutant le produit du gène de la tumeur de Wilms selon la revendication 13 à des cellules malades par la technologie de transfert de gène.

15. Vecteur contenant un segment nucléotidique contenant :
(a) un nombre suffisant de nucléotides correspondant à un gène humain fonctionnel de la tumeur de Wilms tel que défini dans la revendication 1 ou un fragment fonctionnel de celui-ci pour exprimer une protéine ayant une activité de suppression de la tumeur de Wilms, le vecteur ne contenant pas un chromosome humain entier ; et
(b) un promoteur en amont du segment nucléotidique.

16. Vecteur selon la revendication 15 dans lequel le promoteur est un promoteur viral et le vecteur contient également une séquence enhancer et des séquences de polyadénylation.

17. Méthode de quantification de l'ADN de la tumeur de Wilms dans une cellule, comprenant les étapes consistant à :
(a) rendre l'ADN présent dans la cellule disponible pour l'hybridation avec l'ADN complémentaire ;
(b) mettre en contact l'ADN produit à l'étape (a) avec une sonde ADN marquée de manière détectable qui est une séquence nucléoditique complémentaire de l'ADN entier ou d'une portion de celui-ci de la tumeur de Wilms, la séquence nucléotidique dudit ADN ou de la portion de celui-ci étant toute la séquence nucléotidique présentée à la figure 3 ou une portion de celle-ci, et une séquence nucléotidique essentiellement homologue, dans des conditions appropriées pour l'hybridation des séquences d'ADN complémentaire et pour la formation de complexes (ADN de la tumeur de Wilms)/(sonde ADN marquée de manière détectable) ; et
(c) déterminer la quantité de complexes (ADN de la tumeur de Wilms)/(sonde ADN marquée de manière détectable).

18. Sonde ADN pour une utilisation dans la méthode de la revendication 17, (a) qui est complémentaire de la séquence nucléotidique de la revendication 3 ou une portion de celle-ci ; ou (b) consistant essentiellement en la séquence nucléotidique de la figure 3, ou une portion de celle-ci.

19. Méthode de détection d'un polypeptide codé par le gène de la tumeur de Wilms dans un échantillon obtenu à partir d'un individu, comprenant les étapes consistant à :
(a) combiner l'échantillon avec au moins un anticorps spécifique du polypeptide qui a toute la séquence d'acides aminés de la figure 3 ou une portion de celle-ci, dans des conditions appropriées pour la liaison du polypeptide codé par le gène de la tumeur de Wilms et de l'anticorps, et pour la formation de complexes (polypeptide codé par le gène de la tumeur de Wilms)/(anticorps) ; et
(b) détecter les complexes (polypeptide codé par le gène de la tumeur de Wilms)/(anticorps).

20. Méthode selon la revendication 19 comprenant en outre la quantification des complexes (polypeptide codé par le gène de la tumeur de Wilms)(anticorps), par la détermination de la quantité d'anticorps marqués de manière détectable présent dans ledit complexe.

21. Kit pour la détection du gène de la tumeur de Wilms tel que défini dans la revendication 1, comprenant :
(a) une sonde ADN qui est une séquence nucléotidique complémentaire au gène entier de la tumeur de Wilms ou à une portion de celui-ci ; et
(b) un récipient.

22. Kit pour la détection d'un produit polypeptidique codé par le gène de la tumeur de Wilms dans une cellule, comprenant :
(a) un anticorps isolé spécifique d'un produit polypeptidique tel que défini dans la revendication 2 ; et
(b) un récipient.
